(19) 

Europäisches
Patentamt
European
Patent Office
Office européen
des brevets

(11) **EP 1 759 691 A1**

(12) **EUROPEAN PATENT APPLICATION**

(43) Date of publication:
**07.03.2007 Bulletin 2007/10**

(51) Int Cl.:
*A61K 9/127* (2006.01)

(21) Application number: **06022165.2**

(22) Date of filing: **22.11.2004**

(84) Designated Contracting States:
**AT BE BG CH CY CZ DE DK EE ES FI FR GB GR
HU IE IS IT LI LU MC NL PL PT RO SE SI SK TR**
Designated Extension States:
**AL HR LT LV MK YU**

(30) Priority: **20.11.2003 US 523316 P**

(62) Document number(s) of the earlier application(s) in
accordance with Art. 76 EPC:
**04818742.1 / 1 689 364**

(71) Applicant: **Delex Therapeutics Inc.
Mississauga ON L5N 2M2 (CA)**

(72) Inventors:
- **Pliura, Diana Helen
  Mississauga
  Ontario L5M 5A2 (CA)**

- **Ristevski, Blagoja
  Oakville
  Ontario L6M 3P1 (CA)**
- **Boylan, James Charles
  Gurnee
  Illinois 60013 (US)**
- **Li, Hong
  Brampton
  Ontario L6X 4V4 (CA)**

(74) Representative: **Beyer, Andreas
  Wuesthoff & Wuesthoff
  Patent- und Rechtsanwälte
  Schweigerstrasse 2
  81541 München (DE)**

Remarks:
This application was filed on 13 - 11 - 2006 as a
divisional application to the application mentioned
under INID code 62.

(54) **Stable liposome compositions**

(57) Stable liposome composition for delivering a pharmaceutical agent, the composition comprising: (a) a suitable aqueous medium; (b) liposomes formed from a suitable phospholipid; (c) at least one pharmaceutical agent being at least partially encapsulated in the liposomes, and being selected from: (i) a lipophilic amine and a pharmaceutically acceptable acid, wherein the pharmaceutically acceptable acid is selected from an organic or inorganic acid, and (ii) a pharmaceutically acceptable organic acid salt of a lipophilic amine, and optionally a pharmaceutically acceptable organic acid; wherein the quantity of the pharmaceutically acceptable acid present in the composition is such that the pH of the liposome composition is less than or approximately equal to the pKa of the amino group of the pharmaceutically active lipophilic amine. Compositions, kits, and methods for the preparation of same, as well as methods for further enhancing the stability of the compositions by autoclaving, and methods for the identification of stable liposome compositions of the present invention are likewise provided.

EP 1 759 691 A1

**Description**

The invention relates to compositions based on membrane lipids and more specifically to stable liposomes used to deliver a drug and methods for making and using them, even more specifically to sterile and stable liposome compositions used to deliver a drug and methods for making and using them.

**BACKGROUND OF THE INVENTION**

Liposomes, also known as lipid vesicles, are completely closed lipid bilayer membranes which contain an entrapped volume comprising an aqueous medium. The lipid bilayer is often composed of phospholipids such as lecithin and related materials such as glycolipids. Liposomes may be unilamellar, having a single membrane bilayer, or multilamellar, having more than one membrane bilayer and having an aqueous space between different membrane bilayers. The bilayer is composed of two lipid monolayers, each having a hydrophobic portion that is oriented towards each other with the hydrophilic portion facing outwards towards the aqueous phases.

Liposomes are formed when phospholipids or other suitable amphipathic molecules are allowed to swell in water or aqueous solution. If water-soluble materials are included in the aqueous phase during this process, the material may become trapped in the aqueous phase between the lipid bilayers. Similarly, lipophilic materials may be dissolved in the lipid and be incorporated into the bilayers themselves, although if the lipophilic material also has a polar group, this group may extend into the inner or outer aqueous phase. The encapsulation of materials into liposomes can be accomplished by a number of methods known in the art. The method most commonly used involves casting a thin film of phospholipid onto the wall of a flask by evaporation of an organic solvent. When this film is dispersed in a suitable aqueous medium, liposomes are formed. Alternatively, liposomes may also be formed by suspending a suitable lipid in an aqueous medium. The mixture is then sonicated (agitated by high frequency sound waves) to give a dispersion of closed vesicles. A further method for creating liposomes is the rapid mixing of a lipid in ethanol and water. This is often accomplished by injecting the lipid into an aqueous solution.

The lipid bilayer membrane often functions in a manner similar to cell membranes. They therefore exhibit some biological properties such as the ability to be easily accepted into the environment of living cells. Liposomes may merge with living cells as if they were themselves organelles. As a result, there has been much interest in recent years in using liposomes as carriers to deliver compounds possessing a particular biological or pharmaceutical property to a patient.

Some difficulties have arisen in relation to using liposomes as a delivery and targeting means for drugs and other compounds. One particular problem is that liposomes made by conventional techniques with conventional formulations are often phase unstable over time, which can render such compositions unsuitable in industry, in particular the pharmaceutical industry. This can result in the liposomes leaking, breaking down, settling and phase separating, fusing, agglomerating or gellifying upon storage.

Known techniques for increasing the storage stability of liposomes include the use of chemical additives as stabilizers, or the preparation of dry powder preparations. Examples of methods for stabilizing liposomes using excipient stabilizers include those disclosed in U.S. Patent No. 4,818,537, U.S. Patent No.5,100,662, U.S. Patent No. 5,204,112, U.S. Patent No.4,804,539, and U.S. Patent No. 5,962,015. Stabilizers for liposomes have included amphoteric molecules having a cationic region, for example triethanolamine, a common cosmetic buffer. These molecules can be added to prevent aggregation of liposomes. Regardless, triethanolamine has not been shown to provide adequate shelf-life and processing stability. Quaternized alkylated polymers, such as steardimonium hydroxycellulose, have been used to stabilize lecithin-type liposomes containing biologically active ingredients that are acidic. Relatively long chain alkyl amines, such as stearylamine, have also been used to stabilize liposomes but these charged long chain alkyl amines tend to be toxic at elevated levels thus making them less desirable for pharmaceutical applications.

Other techniques used to stabilize liposomes include lyophilization or spray-drying strategies. These steps increase the complexity of manufacture and require reconstitution of the liposomes prior to administration to patients. Reconstitution is not desirable for products that can ultimately be used in the outpatient setting.

One particular area that may be suitable for liposome delivery is inhaled pharmaceutical products. Many inhaled drugs are delivered by way of an aqueous solution that is released from a device capable of generating aqueous aerosol droplets. Regulatory requirements for inhaled pharmaceutical products require that the products be provided as sterile formulations. Previously, strategies for sterilization of liposome formulations have focused on sterile filtration or addition of preservatives. Therapeutic liposomal formulations for delivery by parenteral (iv, im, sc) or inhalation (pulmonary or nasal) routes must be sterile preparations.

Terminal sterlization of pharmaceutical solutions through autoclaving is well established. However, historically liposomes have been noted to be unstable to the harsh conditions of autoclaving leading to agglomeration, fusion, and gellifying of liposomes in much the same manner as observed during storage, as well as change in liposome size or size distribution, hydrolysis/oxidation of lipids, chemical degradation and undesired release of the encapsulated drug, for example as described at page 11, lines 6-10 of WO 2004/00246.

Various groups in the past have attempted to deal with the stability issues associated with autoclaving liposome compositions. For example US Patent 5,554,382 and US Patent 5,776,486 both of which relate to methods for the sterile manufacture of liposomes, teach that liposomes are difficult to sterilize and that sterility is accomplished by independently sterilizing the component parts - lipids, buffer, drug and water - by autoclave or filtration and then mixing in a sterile environment. The patents teach that heat sterilization of the finished product is not possible since heating of liposomes does irreparable damage to liposomes.

US Patent 5,542,935 teaches that heat sterilization of the final liposome products is not possible since heating liposomes does irreparable damage to liposomes but teaches that gaseous precursor-filled multilamellar lipid suspensions may be autoclaved. The autoclaving did not change the size of the gaseous lipid particles.

US Patent 5,770,222, US Patent 6,071,495 and US Patent 6,479,034 also teach the autoclaving of precursor liposomes - i.e. liposomes without a drug payload.

US Patent 5,834,025 provides that ester based liposomes cannot be autoclaved and also discloses that liposomes made with ether lipids obtained from archaeobacteria can be autoclaved. The safety of ester based liposomes, e.g. phosphatidylcholine, is well established whilst the toxicity profile of ether lipids is unknown.

US Patent 5,230,899 describes the autoclaving of preliposome gels containing very little moisture, namely only enough water to be present in an amount of up to 300 moles relative to the solids. Likewise, U.S. Patent 6,424,857 describes the autoclaving of small (100 nm diameter) empty liposomes (i.e. no drug) with no change in particle size reported.

U.S. Patent 5,676,928 describes the autoclaving of multi-lamellar liposomes that have been stabilized by the inclusion of charged phospholipids. The invention is for a diagnostic composition that includes at least one imaging contrast agent.

Overall, the prior art teaches away from using heat as the terminal sterilization step of a finished, drug containing liposomal preparation, and does not teach or suggest an enhancement in phase stability upon terminal sterilization of the liposomes.

Consequently, sterility and pyrogenicity of liposomal preparations has generally been limited to the use of filtration through 0.2 micron filters for preparations containing liposomes below 0.2 microns in diameter or the application of aseptic conditions for manufacture of larger liposomes. Gamma irradiation is viewed as unacceptable due to the unacceptable degradation of the aqueous liposome dispersions. (see, for example, Daan Crommelin, Liposomes as Pharmaceutical Dosage Forms, Encyclopedia of Pharmaceutical Technology, Vol 9, page 13).

Thus, sterile filtration is an option only if the liposomes are sufficiently small to pass through a 0.2 micron filter system. If the objective of liposome formulations is to increase the residence time of a drug at a given site of action, larger multilamellar liposomes are desirable. Liposomes of 1 to 5 microns are suitable for pulmonary delivery, but obviously are not suitable for terminal sterile filtration.

In the past, preservatives and bacteriostatic agents have been added to pulmonary inhalation products. When included in bronchodilator formulations, many of these preservatives have proven to induce pulmonary constriction and counter the beneficial effects of the bronchodilator. Accordingly, addition of preservatives to pulmonary liposome formulations should be undertaken only with caution and is not considered desirable.

Another particular area that may be suitable for liposome delivery is for sustained release compositions of one or more active ingredients, such as those formulations disclosed in US RE38,407 of Delex Therapeutics, Inc. Such compositions can afford rapid onset of the drug from a non-encapsulated portion, followed by sustained release from continued release of liposome-encapsulated active agent. Regulatory requirements for such compositions likewise require that the percent of encapsulated drug be consistent over time and that the formulations are chemically and phase stable over time.

There is therefore a general need to develop liposome formulations with improved stability, in particular the preparation of sterile and stable liposome formulations of drug products that are phase stable and chemically stable and therefore suitable for pharmaceutical use.

## SUMMARY OF THE INVENTION

Included in the scope of the invention are stable liposome compositions and processes for their preparation that are phase stable. A stable liposome preparation for the purpose of the present invention is considered one in which the dispersed liposomes substantially retain their initial character and remain substantially uniformly distributed throughout the continuous phase for the desired shelf-life. Stable liposome compositions of the present invention do not exhibit phase changes, sedimentation and, when they are sterilized via autoclaving, microbial contamination. Stable liposome compositions of the present invention show minimal chemical degradation due to oxidation or hydrolysis of the liposome constituents or the encapsulated drug ingredient.

Therefore, in one aspect of the present invention is provided a stable liposome composition for delivering a pharmaceutical agent, the composition comprising: (a) a suitable aqueous medium; (b) liposomes formed from a suitable phospholipid; (c) at least one pharmaceutical agent being at least partially encapsulated in the liposomes, and being selected from: (i) a lipophilic amine and a pharmaceutically acceptable acid, wherein the pharmaceutically acceptable acid is selected from an organic or inorganic acid, and (ii) a pharmaceutically acceptable organic acid salt of a lipophilic amine, and optionally a pharmaceutically acceptable acid comprising a pharmaceutically acceptable organic acid; wherein the quantity of the pharmaceutically acceptable acid present in the composition is such that the pH of the liposome composition is less than or approximately equal to the $pK_a$ of the amino group of the pharmaceutically active lipophilic amine. In some embodiments of the present invention, the compositions are autoclaved, thereby providing both sterile and stable liposome compositions.

In one aspect of the compositions of the present invention, the pH of the liposome composition is about equal to the $pK_a$ of the amino group of the lipophilic amine, and about 50% of the lipophilic amine is protonated in the composition. In yet another aspect, the pH of the liposome composition is less than the $pK_a$ of the amino group of the lipophilic amine, and a major portion of the lipophilic amine is protonated in the composition, or the composition has a pH of about 1 to about 2 pH units below the $pK_a$ of the amino group of the lipophilic amine. In yet another aspect of the present invention, the pH of the liposome composition is between about 4 and the $pK_a$ of the amino group of the lipophilic amine. In some embodiments, the pH is between about 4 to about 7, or between about 4.5 and about 6, or alternatively between 5 and about 6.

In yet another aspect of the present invention, the compositions further comprises cholesterol and/or ethanol. In one aspect of the present invention, ethanol is present at between about 2.5 % and about 10% of the total volume of the liposome composition.

In yet another aspect of the present invention, the phospholipid of the liposome compositions of the present invention has a net neutral charge at about physiological pH. In one aspect of the present invention, the phospholipid comprises phosphatidylcholine.

In yet another aspect of the present invention, the aqueous medium of the composition comprises water.

In yet another aspect of the present invention, the pharmaceutical agent is both encapsulated in the liposome particles and is also free in the aqueous medium in the compositions of the present invention. In some embodiments, the percent of liposome encapsulated pharmaceutical agent comprises about 50% to about 90% of the total amount of pharmaceutical agent present in the liposome compositions, or about 60% to about 80% of the total amount of pharmaceutical agent present in the liposome compositions, or about 50% to about 75% of the total amount of pharmaceutical agent present in the liposome composition.

In yet another aspect of the present invention, the pharmaceutically acceptable acid of the liposome compositions comprises an organic acid, or an inorganic acid.

In yet another aspect of the present invention liposome particles of the liposome composition have a mass median diameter (d(0.5)) of less than about 10 microns. In some embodiments, the mass median diameter is less than about 6 microns, or about 4 microns, or about 2 microns.

In yet another aspect of the present invention, the autoclaved liposome compositions are physically and chemically stable for at least about one year, or 18 months or two years at a temperature above the freezing point of the liposome compositions.

In yet another aspect of the present invention, the lipophilic amine comprises a lipophilic amine that has a log P value of greater than about 1.0 at physiological pH. In some embodiments, the lipophilic amine has a log P value of between about 2 and about 5 at physiological pH.

In an aspect of the present invention, some embodiments of the liposome compositions are physically and chemically stable to autoclaving, including autoclaving under an inert atmosphere, such as autoclaving at a temperature of about 121°C for a minimum of about 15 minutes under an inert atmosphere.

In yet another aspect of the present invention the ratio of pharmaceutical agent to phospholipid present in the compositions is about between 1:100 and 1:10 mol/mol. The amount of phospholipids present may also be about 1.5 mM or more in compositions of the present invention. In compositions of the present invention, the percent encapsulation of drug in the liposome composition may be substantially stable over a period of at least 20 months. Also compositions of the present invention may be substantially chemically stable over a period of at least 20 months upon autoclaving, wherein the amount of phospholipid does not decrease due to chemical hydrolysis or oxidation by more than 10% (weight/ weight) or more than 5% over a period of at least 20 months. In some autoclaved compositions, the amount of phospholipid does not decrease by more than about 3mg/ml of liposomal composition over a period of at least 20 months. Likewise, in autoclaved compositions of the present invention, the lipophilic amine does not chemically degrade by more than 5% (weight/weight), or 2% over a period of at least 20 months.

In yet another aspect of the present invention is provided sterile and stable liposome compositions for delivering a pharmaceutical agent, the compositions comprising: (a) a suitable aqueous medium; (b) liposomes formed from a suitable phospholipid; (c) at least one pharmaceutical agent being at least partially encapsulated in the liposomes, and being selected from: (i) a lipophilic amine and a pharmaceutically acceptable acid, wherein the pharmaceutically acceptable acid is selected from an organic or inorganic acid, and (ii) a pharmaceutically acceptable organic acid salt of a lipophilic amine, and optionally a pharmaceutically acceptable acid comprising a pharmaceutically acceptable organic acid; wherein the composition is autoclaved, in some embodiments under an inert atmosphere, and wherein the quantity of the pharmaceutically acceptable acid present in the composition is such that the pH of the liposome composition is less than or approximately equal to the $pK_a$ of the amino group of the pharmaceutically active lipophilic amine.

In yet another aspect of the present invention are provided a method for producing the stable liposome compositions of the present invention for delivering a pharmaceutical agent, the method comprising the steps of: (a) providing a suitable aqueous medium; (b) providing a suitable phospholipid; (c) providing at least one pharmaceutical agent being capable of being at least partially encapsulated in the liposomes, and being selected from (i) a lipophilic amine and a pharmaceutically acceptable acid, wherein the pharmaceutically acceptable acid is selected from an organic or inorganic acid, and (ii) a pharmaceutically acceptable organic acid salt of a lipophilic amine, and optionally a pharmaceutically acceptable acid comprising a pharmaceutically acceptable organic acid; wherein quantity of the pharmaceutically acceptable acid present in the composition is such that the pH of the liposome composition is less than or approximately equal to the $pK_a$ of the amino group of the pharmaceutically active lipophilic amine; (d) combining the aqueous medium, phospholipid and pharmaceutical agent to form the liposome composition; and (e) optionally autoclaving said composition. In an important aspect of the present invention, the method includes the step of autoclaving.

In yet another aspect of the present invention are provided the sterile and stable liposome compositions of the present invention, exhibiting one or more of the following characteristics over a period of at least one year upon autoclaving and storage at a temperature above the freezing point of the composition: (i) a change in percent encapsulation of no more than about 5%; (ii) a change in phospholipid content of no more than about 10% by weight; (iii) a change in content of lipophilic amine due to chemical hydrolysis and/or oxidation of no more than about 5% by weight; (iv) a lack of formation of visible aggregates; and (v) a change in the median particle size diameter of no more than about 10% as determined optically. Methods for determining these parameters are detailed below.

In yet another aspect of the present invention are provided stable liposome compositions when prepared by the methods disclosed herein.

In yet another aspect of the present invention are provided stable and sterilized liposome compositions when prepared by the methods disclosed herein.

In yet another aspect of the present invention is provided a method of increasing the stability of liposome compositions, said method comprising the steps of: (a) providing a suitable aqueous medium; (b) providing a suitable phospholipid; (c) providing at least one pharmaceutical agent being capable of being at least partially encapsulated in the liposomes, and being selected from: (i) a lipophilic amine and a pharmaceutically acceptable acid, wherein the pharmaceutically acceptable acid is selected from an organic or inorganic acid, and (ii) a pharmaceutically acceptable organic acid salt of a lipophilic amine, and optionally a pharmaceutically acceptable acid comprising a pharmaceutically acceptable organic acid; wherein quantity of the pharmaceutically acceptable acid present in the composition is such that the pH of the liposome composition is less than or approximately equal to the $pK_a$ of the amino group of the pharmaceutically active lipophilic amine; (d) combining the aqueous medium, phospholipid and pharmaceutical agent to form the liposome composition; and (e) autoclaving said liposome composition at conditions effective to sterilize said compositions, thereby affording compositions with increased stability relative to the stability prior to autoclaving.

In yet another aspect of the present invention is provided a method of identifying a phase stable liposome composition, the method comprising the steps of: (a) providing a liposome composition containing a pharmaceutical agent, phospholipid, aqueous solution, and optionally ethanol and a sterol; (b) optically determining the mass median diameter d(0.5) of the liposome composition; (c) centrifuging the liposome composition at a g-force of between about between about 1000g and about 5000g, at about 4°C for about 2 hours; (d) optically determining the mass median diameter d(0.5) of the any remaining liquid portion of the liposome composition solution after centrifugation step (c); and (e) calculating the ratio of the mass median diameter d(0.5) value of the solution after centrifugation to that of the solution prior to centrifugation; wherein the phase stable liposome composition is identified as such if the composition has a ratio in step (e) of about 0.6 or greater, in some embodiments, 0.8 or greater. In a preferred embodiment, the composition is autoclaved prior to centrifugation.

The compositions of the present invention are particularly suited to pulmonary delivery of pharmaceutical products. In some embodiments of the invention, the composition further comprises at least one of cholesterol and ethanol. In some embodiments of the invention, the compositions are stable to autoclaving.

One advantage of the present invention is that the compositions are stable and do not fuse, separate out, precipitate, agglomerate or gellify upon storage. They therefore remain homogeneous compositions for at least a week and often much longer, in some embodiments, for more than 12 months, or more than 18 months, or more than two years even. The shelf-life of such compositions is therefore increased. Further, this allows unit doses to be apportioned from a larger batch evenly and does not require reconstitution of the composition prior to allocation.

Extended phase stability is one of the formulation features that provides for a pharmaceutically relevant formulation. If the formulation is phase stable for extended periods of time it provides numerous advantages. Phase stable liposome compositions of the present invention makes the final filling process of the final pharmaceutical preparations more robust, and final filling of the pharmaceutical formulations is not constrained by concerns that product will settle or separate before or during filling. Also, phase stable liposome compositions of the present invention provide content uniformity between different individual vials filled from the same batch. Also, individual samplings from the final pharmaceutical container containing the phase stable pharmaceutical formulations of the present invention will be more uniform from dose to dose, thereby providing improved safety for the patient, without requiring that the patient fully shake the composition prior to use. This may be particularly advantageous for elderly patients. If the liposome encapsulated drug compositions phase separate, there is a possibility that a patient may receive too high a dose if the sample is taken from the more dense layer or sediment of the formulation, or that a patient may be under-dosed if the sample is taken from a less dense layer, or the patient may receive no effective dose if the sample is withdrawn from a clear supernatant. The present invention provides compositions and methods that afford confidence in dose to dose reproducibility.

The compositions of the present invention do not require chemical additives in order to be stable. Liposomes of the invention comprise a lipophilic amine and an acid, such as an organic acid, wherein the acid is present in a concentration such that the pH of the liposome composition is about equal to or less than the $pK_a$ value of the amino group of the lipophilic amine, provided that the pH of the final solution does not compromise the chemical stability of the liposome compositions, typically seen below about pH 4. Being at a pH that is about equal to or less than the $pK_a$ ensures that at least a substantial portion of the lipophilic amine is positively charged in the liposome compositions. In one aspect of the invention, the pharmaceutical agent comprises a lipophlic amine and an organic acid as a counter-ion, for example, fentayl citrate. This may be provided in the compositions by combining free base fentanyl dissolved in an ethanol phase, with citric acid in an aqueous phase, and the phases, along with the other components are combined together to afford fentanyl citrate within the meaning of the present invention. Alternatively, for example, the salt form of the drug fentanyl, such as fentayl citrate, may be commercially purchased as used in the preparation of the liposome compositions. In some instances, it may be further required to add additional amounts of an acid, such as citric acid, to the compositions in order to adjust the pH of the liposome compositions, to afford a pH that is about equal to or below the $pK_a$ of the amino group of the lipophlic amine, provided that the pH of the liposome solutions is not below about pH 4, where chemical stability of the compositions is typically comprised. The ability to stabilize liposomes without a further chemical additive is particularly desirable for compositions intended for administration to a patient since it removes the risk of side affects resulting from the chemical additive.

In embodiments of the present invention, the liposome compositions are further stable upon autoclaving. The ability to autoclave the liposomal compositions of the invention provides an easy method to sterilize the formulations. Unlike filtration, autoclavable liposome compositions will allow for larger liposomes and thus a higher drug encapsulation. Furthermore, the ability to autoclave liposome compositions obviates the need to add preservatives or bacteriostatic agents which are generally undesirable, particularly for pulmonary formulations.

According to a further aspect of the invention, a method for using the liposomal compositions as a medicament is provided.

According to a further aspect of the invention, the stable liposomal compositions are included in packaged pharmaceuticals and kits along with a device for use in pulmonary delivery of therapeutic agents that is capable of generating aqueous aerosol droplets of the stable liposome compositions.

In another aspect of the present invention is a stable liposome composition and a method for the production thereof, and use thereof, wherein the phospholipid comprises phosphatidylcholine.

**BRIEF DESCRIPTION OF THE DRAWINGS**

These and other features of the invention will become more apparent in the following detailed description in which reference is made to the appended drawings wherein:

**Figure 1** shows a table summarizing various liposomal compositions and the results of the visual analysis of phase stability both before and after autoclaving the compositions;

**Figure 2** shows a comparison of appearance of a phase stable liposome preparation of the present invention **(Figure 2a)** with the appearance of a liposome preparation that separates following preparation and storage **(Figure 2b);**

**Figure 3** shows the phase stability of placebo liposomes lacking a lipophilic amine pharmaceutical agent as a function of pH of the formulation **(Fig. 3a);** and the stabilizing effect of a lipophilic amine on the compositions as a function of pH **(Fig. 3b);**

**Figure 4** shows a table summary of various liposome preparations, the pH, the particle size (d(0.5.)) before and after autoclaving, and the phase stability index before and after autoclaving;

**Figure 5** shows a graph illustrating the relationship between pH after autoclaving versus pH before autoclaving for liposome compositions of the present invention;

**Figure 6** shows a graph plotting the % change in phosphatidylcholine content after autoclaving for various liposome compositions compared to the phosphatidylcholine content before autoclaving, as a function of pH;

**Figure 7** shows a graph plotting the change in particle size distribution after autoclaving of various liposome compositions relative to the particle size distribution of the compositions before autoclaving as a function of pH;

**Figures 8a-8c** show photographs of an unstable liposome composition, a liposome composition having intermediate stability, and a stable liposome composition of the present invention, respectively;

**Figure 9** shows a photograph of a liposome composition having intermediate stability;

**Figure 10** shows a graph plotting the phase stability index of the fentanyl compositions of **Figure 4,** as a function of pH, pre-autoclaving;

**Figure 11** shows a graph potting the phase stability index of the compositions containing fentanyl of **Figure 10**, as a function of pH, post-autoclaving;

**Figure 12** shows a graph plotting the phase stability index of liposome compositions containing ondansetron of **Figure 4,** as a function of pH, pre-autoclaving;

**Figure 13** shows a graph potting the phase stability index of the liposome compositions containing ondansetron of **Figure 12,** as a function of pH, post-autoclaving;

**Figure 14** shows a graph plotting the phase stability index of the liposome compositions containing ondansetron of **Figure 4,** as a function of pH, pre-autoclaving, and excluding formulations containing palmitic acid as the organic acid and formulations with 2.4 mmol ondansetron;

**Figure 15** shows a graph potting the phase stability index of the liposome compositions containing ondansetron of **Figure 14** as a function of pH, post-autoclaving; and excluding formulations containing palmitic acid as the organic acid and formulations with 2.4 mmol ondansetron;

**Figure 16** shows a graph plotting the phase stability of the liposome compositions containing sumatriptan of **Figure 4,** as a function of pH, pre-autoclaving;

**Figure 17** shows a graph potting the phase stability index of the liposome compositions containing sumatriptan of **Figure 16**, as a function of pH, post-autoclaving;

**Figure 18** shows a graph plotting the phase stability index of the liposome compositions containing prochlorperazine of **Figure 4,** as a function of pH, pre-autoclaving;

**Figure 19** shows a graph potting the phase stability index of the liposome compositions containing prochlorperazine of **Figure 18**, as a function of pH, post-autoclaving.

## DETAILED DESCRIPTION OF EMBODIMENTS

In the following description, numerous specific details are set forth to provide a thorough understanding of the invention. However, it is understood that the invention may be practiced without these specific details.

The methods of the present invention are claimed and described herein as a series of steps. It should be understood that these methods and associated steps may be performed in any logical order. Moreover, the methods may be performed alone, or in conjunction with other procedures and treatments administered before, during or after such methods and steps set forth herein without departing from the scope and spirit of the invention.

## Liposome Compositions Components

The present invention provides liposome compositions that have a pharmaceutical agent incorporated in the liposomes and are phase stable for an extended period of time. Liposomes compositions of the present invention comprise a lipophilic amine and an acid, such as an organic acid, wherein the acid is present in a concentration such that the pH of the liposome composition is about equal to or less than the $pK_a$ value of the amino group of the lipophilic amine, provided that the pH of the final solution does not compromise the chemical stability of the liposome compositions, typically seen below about pH 4. Being at a pH that is about equal to or less than the $pK_a$ ensures that at least a substantial portion of the lipophilic amine is positively charged in the liposome compositions. In some embodiments of the invention, the pharmaceutical agent may already be a salt of a lipophilic amine and an acceptable acid, preferably an organic acid. Compositions according to the invention are phase stable on storage for at least one week and in most instances much longer, preferably one year or more.

In some embodiments, the pharmaceutical agent comprises a lipophlic amine and an organic acid as a counterion, for example, fentanyl citrate. This may be provided in the compositions by combining free base fentanyl dissolved in an ethanol phase, with citric acid in an aqueous phase, and the phases, along with the other components are combined together to afford fentanyl citrate within the meaning of the present invention. Alternatively, for example, the salt form of the drug fentanyl, such as fentayl citrate, may be commercially purchased as used in the preparation of the liposome compositions. In some instances, it may be further required to add additional amounts of an acid, such as citric acid, to the compositions in order to adjust the pH of the liposome compositions, to afford a pH that is about equal to or below the $pK_a$ of the amino group of the lipophlic amine, provided that the pH of the liposome solutions is not below about pH 4, where chemical stability of the compositions may be comprised.

Liposome compositions of the present invention contain liposomes formed by closed bilayers of phospholipids. Suitable phospholipids for forming liposomes intended to deliver pharmaceutical agents are known in the art and include, but are not limited to, phosphatidic acid (PA) and phosphatidyl glycerol (PG), phosphatidylcholine (PC), phosphatidylethanolamine (PE), phosphatidylinositol (PI), phosphatidylserine (PS), plasmalogens, and sphingomyelin (SM). The lipids used to form the liposome compositions of the present invention may be either charged or neutral. In one embodiment, liposomes formed from phosphatidylcholine are preferred since they generally are more benign when administered to a patient and pharmaceutical agents often incorporate better into neutral phospholipids. In another embodiment, positively charged liposomes are desirous.

Sterols such as cholesterol are routinely added to liposome compositions to increase stability of the liposomes and promote incorporation of the liposomes into a living environment. The term "cholesterol" is intended to encompass cholesterol derivatives such as: (3-hydroxy-5,6-cholestene) and related analogs, such as 3-amino-5,6-cholestene and 5,6-cholestene; cholestane, cholestanol and related analogs, such as 3-hydroxy-cholestane; and charged cholesterol derivatives such as cholesteryl beta.-alanine and cholesteryl hemisuccinate. In some embodiments, cholesterol is present from about 0% to about 30% of the weight of the phospholipids present. In other embodiments, cholesterol is present up to about 10% of the weight of the phospholipid.

Pharmaceutical agents of the present invention are agent that may be administered to a patient for a therapeutic purpose. The agents of the invention are selected from a group consisting of pharmaceutically active lipophilic amines and their respective salts. Lipophilic amines of the present invention refer to molecules that consist of an organic solvent soluble (lipophlic moiety) as well as an amine moiety that carries a positive charge at physiological pH. Suitable lipohilic amines of the present invention have a positivly charged amino group over the pH range of about 3 to about 8.

Suitable pharmaceutical agents include, but are not limited to the following:

| | | | | |
|---|---|---|---|---|
| Acebutolol | Dihydrocodeine | Isoproterenol | Nalmefene | Propafenone |
| Albuterol | Dihyrdorergotamine | Isoxsuprine | Naloxone | Propoxyphene |
| Alfentanil | Diltiazem | Ketamine | Naltrexone | Propranolol |
| Alosetron | Diphenoxin | Labetalol | Naratriptan | Protriptyline |
| Amitriptyline | Disopyramide | Leuprolide | Nefazadone | Pseudoephedrine |
| Anileridine | Dobutamine | Levamisole | Nifedipine | Quetiapine |
| Atenolol | Dolasetron | Lidocaine | Norepinephrine | Quinidine |
| Atropine | Donepezil | Lisinopril | Nortriptyline | Quinine |
| Azatadine | Dopamine | Lorazepam | Ondansetron | Raloxifene |
| Baclofen | Doxapram | Lovarphanol | Orphenadrine | Reserpine |
| Benztropine | Doxepin | Mafenide | Oxprenolol | Rimantadine |
| Bextolol | Doxylamine | Maprotiline | Oxybutynin | Ritodrine |
| Biperiden | Droperidol | Mazindol | Oxycodone | Ropivacaine |
| Brimonidine | Enalapril | Meclizine | Oxymorphone | Scopolamine |
| Bromocriptine | Ephedrine | Melphalan | Oxytetracycline | Selegiline |
| Bupivacaine | Epinephrine | Meperidine | Palonosetron | Sotalol |
| Buprenorphine | Ergoloid | Mepivacaine | Penbutolol | Sufentanil |
| Butenafine | Ergotamine | Mesoridazine | Pentazocrine | Sumatriptan |
| Butorphanol | Estazolam | Metaproterenol | Pergolide | Tacrine |
| Caffeine | Fenoldapam | Metaraminol | Perphenazine | Tamoxifen |
| Carteolol | Fentanyl | Methacycline | Phenazopyridine | Terbutaline |
| Cefepine | Fexofenadine | Methadone | Phendimetrazine | Tetracycline |
| Cephalexin | Flecainide | Methotrimeprazine | Phenelzine | Thiethylperazine |
| Chloroprocaine | Fluoxetine | Methylamphetamine | Phenoxybenzamine | Thioidazine |
| Chlorpheniramine | Fluphenazine | Methylclothiazide | Phentermine | Thiothixene |
| Chlorquinine | Flurazepam | Methyldopa | Phentolamine | Tocainide |

(continued)

| | | | | |
|---|---|---|---|---|
| Ciprofloxacin | Formoterol | Methylergonovine | Phenylephrine | Tolazoline |
| Citalopram | Glipizide | Methylphenidate | Pimozide | Trandolapril |
| Clomiphene | Haloperidol | Methylsergide | Pinolol | Trazodone |
| Clonidine | Hydralazine | Metoclopramide | Piroxicam | Trifluoperazine |
| Codeine | Hydrocodone | Metolazone | Prazosin | Triflupromazine |
| Cyclobenzaprine | Hydromorphone | Metoprolol | Primaquine | Trihexyphenidyl |
| Demeclocycline | Hydroxychloroquine | Mexiletine | Procainamide | Trimeprazine |
| Desipramine | Hydroxyzine | Midazolam | Procarbazine | Trimethobenzamide |
| Desmopressin | Hyoscyamine | Minocycline | Prochlorperazine | Verapamil |
| Dextroamphetamine | Imipramine | Moricizine | Procyclidine | Zolmitriptan |
| Diazepam | Indapamide | Morphine | Promazine | |
| Diethylpropion | Isoetharine | Moxifloxacin | Promethazine | |

Suitable lipophilic amines of the present invention comprise lipophilic amines that have a log P value of greater than about 1.0 (i.e. a partition coefficient in octanol/water of greater than 10), more preferably between about 2 and about 5 at physiological pH. Lipophilic amines of embodiments of the present invention include fentanyl which is reported to have a log P value of 4.25, ondansetron which has a log P value of 2.37, sumatriptan which has a log P value of 1.05, and prochlorperazine which has a log P value of 3.82.

The acid for use in the present invention is any pharmaceutically acceptable acid. Suitable acids include organic and inorganic acids and include those acids which retain the biological effectiveness and properties of the drug and which are not biologically or otherwise undesirable. Examples of include but are not limited to acetic acid, ascorbic acid, aspartic acid, benzoic acid, butyric acid, carbonic acid, caproic acid, citric acid, cinnamic acid, decanoic acid, enathic acid, fumaric acid, furoic acid, gluconic acid, glucuronic acid, glutamic acid, glyceric acid, hippuric acid, hydrochloric acid, lactic acid, lactobionic acid, mandelic acid, malic acid, maleic acid, methanesulfonic acid, myristic acid, oleic acid, oxalic acid, palmitic acid, pivalic acid, picolinic acid, phosphoric acid, propionic acid, succinic acid, salicylic acid, stearic acid, sulfuric acid, tartaric acid, undecylic acid and valeric acid.

Liposome compositions used to deliver a pharmaceutical agent preferably have a pH that would be physiologically tolerated by the patient. For example, for inhaled compositions, the pH of lung tissue is reported to be from 6.8 to 6.9. The pH of the liposome compositions of the present invention typically have a value of between about pH 4 and about pH 8. In one embodiment, such as in one embodiment where the compositions are autoclaved, the pH of the liposome compositions of the present invention is between about pH 4 and about pH 7. In another embodiment, such as where the compositions are autoclaved, the pH of the liposome compositions of the present invention is between about pH 5 and about pH 6. Although liposome compositions of the present invention may be phase stable at pH values lower than about 4, such formulations are not typically chemically stable, and oxidation and/or hydrolysis of the phospholipid, among other reactions, can occur at lower pH values over time.

In the present invention, the pH of the composition is less than or approximately equal to the $pK_a$ of the amino group of the lipophilic amine active ingredient. In one embodiment, the pH of the solution is between about 1 to 2 pH units below the $pK_a$ of the amino group of the lipophilic amine. Having a pH value that is below the $pK_a$ provides that a major portion of the amino group of the lipophilic amine is positively charged.

Ethanol routinely forms part of liposome compositions, particularly where ethanol is used to prepare the liposomes by routine methods. Alternatives are available, but are limited to those that do not possess unwanted toxicities or are not known to be irritant upon inhalation. Suitable alternatives are glycols and glycerols that meet such requirement. The content of ethanol that may be present in liposome compositions of the present invention can be any which yield the stable liposome compositions of the present invention. In one embodiment, the concentration of ethanol is between about 2.5% and 10 % of the total volume of the liposome compositions. Compositions with ethanol greater than 10% of the volume are also within the context of the present invention, although as concentrations approach or exceed 15% of the total volume, such as at 20%, the quality of the liposome particles formed begins to be compromised.

The aqueous medium of the present invention can be any physiologically acceptable aqueous solution such as buffers or water which do not interfere with the formation of the derived liposome compositions. In one embodiment, the buffer comprises a low ionic strength buffer. It has been shown that for buffers, a sufficiently low ionic strength buffer should be used so as to not effect the efficiency the formed liposomes to encapsulate the pharmaceutical agent. In a preferred embodiment, the aqueous solution is water.

In some cases, additional excipients are added to the liposome compositions of the present invention, such as an anti-oxidant compounds, in some embodiments at about up to 1 or 2% of the phospholipids (weight/weight), typically accounting for 0.01 - 0.1% of the total volume of the composition. Additional components that may be added to the compositions of the present invention include only those that do not effect the phase stability liposome compositions, and thereby do not compromise the robustness of the compositions. In a preferred embodiment, the compositions consist essentially of a pharmaceutical agent including an acid, a phospholipid, an aqueous solution, and optionally ethanol and a sterol.

**Preparation of Liposome Compositions**

It will be understood by those skilled in the art that liposome suspensions of the invention can be prepared by standard methods for preparing and sizing liposomes. These include hydration of lipid films, solvent injection, and reverse-phase evaporation.

The liposome compositions in the following specific examples were prepared by mixing an ethanolic phase with an aqueous phase. The ethanolic phase comprised ethanol, the pharmaceutical agent, phosphatidylcholine, and cholesterol. The pharmaceutical agent was selected from a group of drugs consisting of lipophilic amines. The aqueous phase comprised water for injection and a negative counter-ion to the amine provided by an acid. In cases where the pharmaceutical agent is a salt of a lipophilic amine and an acid, the aqueous phase comprised water for injection. In some embodiments, the acid comprises a hydrophobic acid, and this can be added to the ethanolic phase, which is subsequently mixed with the aqueous phase. This may be useful in the preparation of liposome compositions of lipophilic amines with a desired acid, where the amine is not available as the salt form thereof.

The aqueous phase could comprise an additional amount of acid that does not affect phase stability. Both phases before mixing are heated to a temperature of about 56 to 60 degrees centigrade. For small scale preparations of less than 1 litre in volume, the two phases are mixed and placed on an environmental shaker at 75-80 RPM. Liposomal vesicles are formed and the mixture is shaken for a further 10 minutes at 56-60 degrees centigrade. The mixture is then removed from the shaker and allowed to cool to room temperature for approximately two hours. For larger scale preparation of liposome compositions, the ethanolic phase is added to a stirred aqueous phase in a reactor equipped with temperature control features. The mixture is stirred for 10 minutes at about 56-60°C, and then cooled to room temperature over a two hour period. Stable compositions are characterized in that they remain homogeneous and do not phase separate upon storage over a one week period. In general, it has been found that the reagents may be mixed together or added together in any order so long as they afford the liposome compositions.

In some embodiments, the rate of addition of ethanol phase to the aqueous phase has been found to effect particle size distribution of the formed liposomes, particularly when larger volume batches of the liposome are prepared where it is not practical to add the aqueous phase to the ethanol phase. It has been found in some embodiments that the particle size of liposomes is decreased as the rate of additional of ethanol to water is decreased. It is to be understood that the liposome compositions of the present invention include those with a variety of particle size distributions.

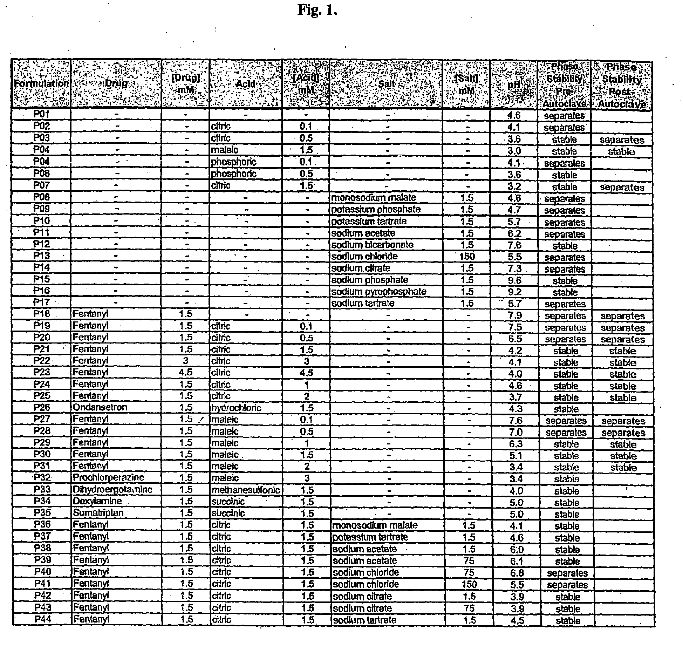

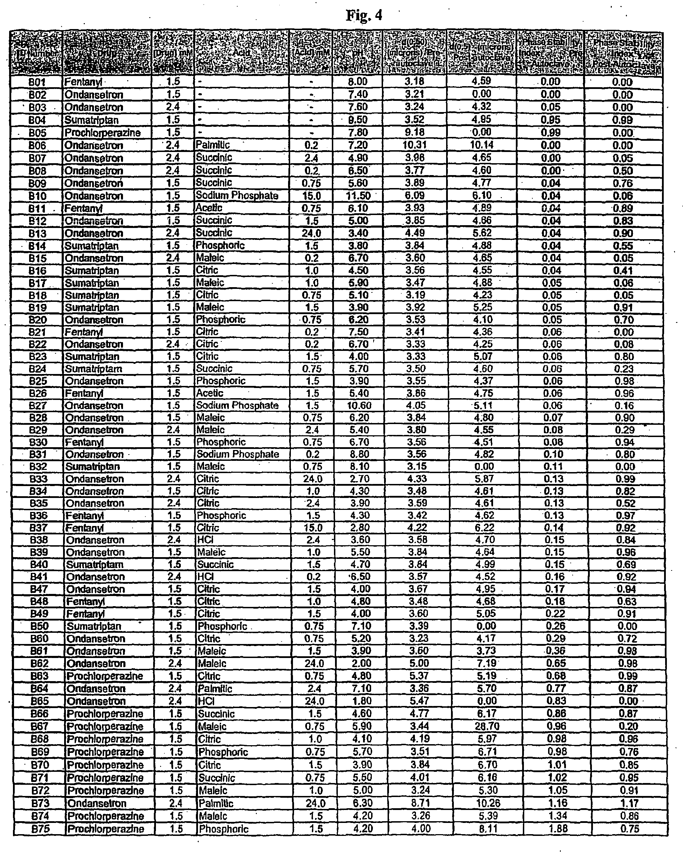

A variety of formulations have been prepared using different lipophilic amines, acids, or salts thereof, as set out in **Figures 1** and **4** in order to illustrate the present invention.

A variety of batch sizes have been prepared using this method, including batches having a total volume of liposome composition as small as 30 ml, as well as larger batches of 1, 2.5, 5 and 15 litres as described in **Example 4**, below. Liposome compositions prepared in both small and large volumes have been found to be suitably stable over time. Studies of liposome compositions made in batches between 1 and 15 litres have been shown to have comparable chemical and physical stability, mass median diameter of liposome particles, and comparable percent encapsulation of the active ingredient.

The pharmaceutical composition of this invention may be administered in numerous ways, including via inhalation through the pulmonary system, topically, parenterally and the like. The compositions may include ophthalmic dosage forms, and injectable dosage forms, and may include medical diagnostic products.

The term "parenteral" as used herein and as is understood to skilled persons in the art (for example, see Stedman's Medical Dictionary, 25th edition, 1990 (Williams & Wilkins)) is meant to include any other route than through the gastrointestinal tract, in particular referring to the introduction of substances into an organism by intravenous, subcutaneous, intramuscular, or intramedullary injection. The pharmaceutical composition may be in the form of a sterile injectable preparation, for example, as a sterile injectable aqueous or oleaginous suspension or inhalation product for administration via nebulization. Given that the formulations are phase stable, they may be used orientation independent by the end user using a variety of devices such as top fed and bottom fed nebulizers.

**Liposome stability -physical and chemical stability**

For the purpose of the present invention, "stable liposome compositions" are those in which the dispersed liposomes substantially retain their initial character and remain substantially uniformly distributed throughout the continuous phase and show minimal chemical degradation for the desired shelf-life.

The term "physical stability" of the liposome compositions as used herein refers to the absence of significant changes in characteristics of the liposomes, such as liposome size (expressed as the mass median diameter d(0.5)), ratio of encapsulated to free drug in the composition, sedimentation, aggregation, or light scattering properties of the compositions over the storage time.

As used herein, the term "particle size distribution" or "PSD" refers to the particle size distribution in a liposomal dispersion as measured by dynamic light scattering techniques which are well known to skilled person in the art, such as with a Malvern Mastersizer™ 2000. A convenient way to report the particle size distribution of the liposome compositions is by reporting the d(0.5.) value which refers to the mass median diameter of the particles and represents the median size of the liposomes, with 50% of the sample associated with smaller liposomes and 50% of the sample associated with larger liposomes. The preferred range of particle size of liposomes of the present invention is less than about 10 microns, preferably less than about 6 microns, or less than about 4 microns, or less than about 2 microns. It will be understood by skilled persons in the art that the desired ranges of particle sizes may vary depending on the application. For example, a d(0.5) value falling within the 1-3 micron range can maximize the percentage of liposomes that fall within the respirable size of liposomes.

The "Percent encapsulation" or encapsulation ratio or %E for various formulations refers to the percentage of lipophilic amine encapsulated within the liposome relative to the total amount of lipophilic amine in the compositions. The percentage encapsulation of the lipophilic amine in the liposomes of the present invention is expressed as the percentage of a pharmaceutical agent incorporated into the liposomes. A preferred range of encapsulation is from about 50% to about 90%, more preferably about 60% to about 80%, more preferably about 50% to about 75%. Skilled persons in the art will appreciate that different percents encapsulation may be advantageous for different applications, depending, inter alia, on the time of initial onset and total period of action of the drug product that is desired.

Placebo liposomes were prepared in the above manner that contained no pharmaceutical agent. These placebo liposomes therefore contained only phospholipid, cholesterol, ethanol, water and in some cases a salt. These liposomes were found to be unstable and underwent phase separation within hours to days of preparation.

Liposomes prepared as above but containing the free base of a lipophilic amine drug as the pharmaceutical agent were also unstable upon storage. A pharmaceutically acceptable acid was not added to the compositions. Phase separation was noted within days of preparation.

Liposomes containing the protonated form of the free base of the lipophilic amine base ion and a corresponding acid counter-ion in were found to provide stable liposome preparations. In particular, in some embodiments, molar ratios of acid:amine ranging from about 10:1 to 1:10 (molar/molar) was found to be effective to improve stability of the liposomes. Narrower ratios of acid:amine of between 3:1 and 1:3 are also effective. Additionally, these liposomes were also found to be stable to autoclaving. Liposomes prepared with a pharmaceutical agent, that is the salt of a lipophilic amine and an organic acid, also showed stability on storage and are stable to autoclaving.

Dialysis of the liposome encapsulated drug products under conditions that deplete the liposomes of drug candidate result in the liposome compositions undergoing phase separation. In Table 1 below, various liposome compositions were dialyzed over the noted periods of time. Stable liposome compositions of the present invention with water as the aqueous solvent showed substantially the same percent encapsulation after dialysis as beforehand.

**Table 1**

| Liposome formulation | Dialysis Medium | % E before dialysis | % E after 24 hours dialysis | Appearance after dialysis |
|---|---|---|---|---|
| Fentanyl with Citrate | Water for injection | 60% | 60% | homogenous |
| Fentanyl with Citrate | Phosphate buffer | 60% | <14% | sediment |
| Ondansetron with HCl | Water for injection | 60% | 60% | homogeneous |
| Onsdansetron with HCl | Phosphate buffer | 60% | 11% | sediment |

Surprisingly, autoclaving of the liposome encapsulated drug products of the present invention results in suitably stable and sterile liposome encapsulated drug products. Even more surprisingly, it some cases it was observed that stable liposome encapsulated drug products showed an enhancement of stability relative to the pre-autoclaved stable product. As such, in another aspect of the present invention, there is provided a method of increasing the stability of liposome compositions, wherein the liposome compositions of the present invention are autoclaved under an inert atmosphere. Even if such compositions are not required to be sterile for their applications, such as topical compositions, the step of autoclaving may be used in the method to enhance the stability of the liposomes.

Autoclaving conditions suitable for use with the present invention include those which allows for the sterilization of the liposome encapsulated drug product and which do not result in a substantial decrease in the chemical stability of the compositions over time. In one embodiment, the liposome encapsulated drug products are autoclaved at about 121°C for a minimum of about 15 minutes under an inert atmosphere such as nitrogen or argon. In one embodiment, an inert atmosphere is one that generally contains less than about 1.5% oxygen. Higher temperatures may be likewise used with shorter periods of time. If a formulation can withstand the heating/cooling cycle then terminal sterilization represents a robust, rapid and inexpensive method for the preparation of sterile pharmaceuticals.

Numerous formulations have been prepared using different lipophilic amines, organic acids, or salts thereof, as set out in **Figures 1 and 4** and their stabilities tested to further illustrate the stable compositions and methods for the preparation of the stable compositions of the present invention.

It is a feature of the present invention that the liposome encapsulated drug product compositions constitute homogeneous dispersions. In some compositions, the homogeneous dispersions take on a translucent appearance and do not require shaking prior to administration as they are homogeneously dispersed, despite the tendency of an end-user to include such a step for turbid dosage forms. The stable compositions were observed to be physically and chemically stable over extended periods of time and did not indicate any visible aggregates, in some embodiments, even after 24 months. Unstable liposome-encapsulated drug products show precipitation, separation and aggregation after a period of time.

In the present invention, a stable liposome composition is one that is phase stable and does not substantially form aggregates, preferably ones that do not form aggregates in about at least one year, more preferably in at least about 18 months or more, even more preferably over at least about 24 months, although the periods can be longer or shorter as required, depending upon, *inter alia,* the active ingredient to be delivered and the mode of delivery.

The phase stability of the liposome compositions can be also predicted by a protocol as described in **Example 3** herein, which measures the d(0.5) value of the liposome particles and the obscuration in the supernatant of liposome compositions when centrifuged. Further details are set out in the Examples below.

Furthermore, stable liposome encapsulated drug products of the present invention are characterized by substantially consistent particle size over time during storage, including those stable liposome encapsulated drug products that have been sterilized by way of autoclaving as described herein. For example, **Example 4** below shows the particle size distribution of various liposome compositions of the present invention remained substantially unchanged over periods of time up to 20 months of storage, evidencing that the formulations of the present invention are substantially stable in particle size over storage time. Further details are set out in the examples below.

Furthermore, stable liposome encapsulated drug products of the present invention are characterized by a substantially stable percent encapsulation of the active ingredient over time during storage, including those stable liposome encapsulated drug products that have been sterilized by way of autoclaving as described herein. While autoclaving some compositions of the present invention has been shown to effect the percent encapsulation of the active ingredient relative to the percent encapsulation of active ingredient prior to autoclaving, it is to be understood the difference in percent encapsulation over time as discussed herein refers to the change in percent encapsulation over time of the composition that has not been autoclaved, or during the storage time period subsequent to autoclaving. It is also to be understood that the desired percent of free drug to encapsulated drug can in the present compositions for use can vary, depending upon the nature of the active ingredient, the desired dosage and the relative contribution of the encapsulated and free drug to the desired therapeutic effect.

To evidence the physical stability of the liposomes, **Example 4** below shows the stability of the percent encapsulation of the active ingredient of various liposome compositions of the present invention over time periods of up to 20 months under an inert atmosphere. Further details regarding Example 4 are set out below.

Furthermore, stable liposome compositions of the present invention are characterized by being substantially chemically stable over time. The term "chemical stability" as used herein is used to refer to the absence of significant changes to the chemical structure of the lipophilic amine, acid, phospholipid, cholesterol or other components of the final composition. For the active ingredient, specifically the lipophilic amine, chemical stability is defined as less than about 5% degradation or change in potency of the active ingredient, preferably less than about 2% degradation over the storage period. For the phospholipids carrier, chemical stability is defined as the presence of less than about 10% loss in phopsholipid content due to degradation of the liposome through hydrolysis or oxidation.

In one embodiment, the liposome compositions of the present invention were found to be substantially stable at 4°C, pH 4 for at least one year with some oxygen present. In another embodiment as described in **Example 4** below, the chemical stability of phosphatidylcholine of the liposome compositions of the present invention was found to be substantially unchanged over time periods of up to 20 months. Further details regarding Example 4 are set out below.

The pH of the liposome compositions of the present invention typically have both phase and chemical stability at a pH value of between about pH 4 and about pH 8. In one embodiment, the liposomes of the present invention have chemical and phase stability at a pH of the between about pH 4 and about pH 7. In another embodiment, the liposome composition of the present invention have chemical and phase stability at a pH of between about 4.5 and about 6.5, or about pH 5 and about pH 6. Although liposome compositions of the present invention may be phase stable at pH values lower than about 4, such formulations are not typically chemically stable, and oxidation and/or hydrolysis of the phospholipid, among other reactions, can appreciably occur at such lower pH values over time.

Liposome compositions of the present invention comprise a lipophilic having a charged amino group as an active ingredient that imparts stability to the liposome compositions. It has been observed that liposomes prepared in the absence of a charged lipophilic amine are unstable and rapidly sediment. Phospholipids commonly used in the preparation of liposomes include phosphatidylcholine. While not wishing to be bound by any particular theory, the following provides a description of the interactions and forces that may contribute to the stabilization or destabiliziation of the liposome compositions. At physiological pH ranges, phosphatidylcholine behaves as a neutral molecule with net zero charge. At physiological pH, the negative charge of the phosphatidyl group is balanced by the charge of the quaternary ammonium nitrogen of the choline moiety. Within the liposome, the phsophatidylcholine molecules are generally aligned in a side-by-side manner so that the positively charged choline group of one molecule interacts electrostatically with the phosphatidyl group of an adjacent lipid molecule. The net charge of such a liposomal preparation is zero (as reflected by zeta potential measurements). The inclusion of uncharged drugs into the liposome does not alter the net charge of the liposomes. The stabilization of the liposomes with lipophilic amine drugs is achieved by the selection of pH of the liposome composition such that the pH is about equal to or less than the $pK_a$ of the lipophilic amine, imparting the lipophilic amine with increasingly positive charge. A positively charged lipophilic amine thus may insert within the phospholipid bilayers of the liposome structure in a manner that more closely aligns the positive charge of the amine with the negatively charged phosphatidyl moiety and may disrupt the balance of charge on the surface of the liposome to create a net positively charged liposome at physiological pH. It is believed that being at a pH less than the $pK_a$ of the lipophilic amine thus can enhance the role in the drug as a stabilizer in the liposome formulations per se, by affording a charged liposome at the surface. Charged liposomes may thus repel each other affording resistance to aggregation. This could likewise account for the inability to prepared stable placebo liposome compositions that do not contain a lipophilic amine and its salts thereof, and a pharmaceutically acceptable acid. Furthermore, this could likewise account for the inability to prepare stable liposome compositions with lipophilic amines that are presented predominately as uncharged, neutral molecules.

Thus, in the present invention, the pH of the liposome compositions of the present invention is about equal to or less than the $pK_a$ value of the amino group of the lipophilic amine active ingredient.

It is to be understood that the various ranges as discussed above and used in the context of the present invention in relation to stability are to be understood to be approximate and serve as a guideline, and would be understood by persons of skill in the art to encompass any variants thereof as long as the resultant compositions are stable as defined here.

The advantages of the present invention are further illustrated by the following examples. The examples and their particular details set forth herein are presented for illustration only and should not be construed as a limitation on the claims of the present invention.

### Examples

### Example 1- Preparation of Liposomes

Each test batch of liposome preparation was prepared with 1.2 g purified soya lecithin, 0.12 g of cholesterol dissolved in 3 gm of ethanol and warmed to 56 degrees Celsius. In some preparations, the ethanolic phase also contained the lipophilic amine or a salt of the lipophilic amine (**Figure 1**) in an amount that provided the target concentration in the final formulation. Following dissolution of all constituents in the ethanolic phase, the ethanolic solution was mixed with 27 g of aqueous solution warmed to 56 degrees Celsius. The aqueous phase optionally contained various acids or salts as indicated in **Figure 1.** Following mixing of the two liquid phases, the mixture was shaken at 56 degrees Celsius for 10 minutes on an orbital shaker and then gradually cooled to ambient temperature. The presence of multilamellar liposomes was confirmed by microscopy. Selected liposome preparations were autoclaved at 121 degrees Celsius for 15 minutes.

### Example 2 - Phase Stability on Storage

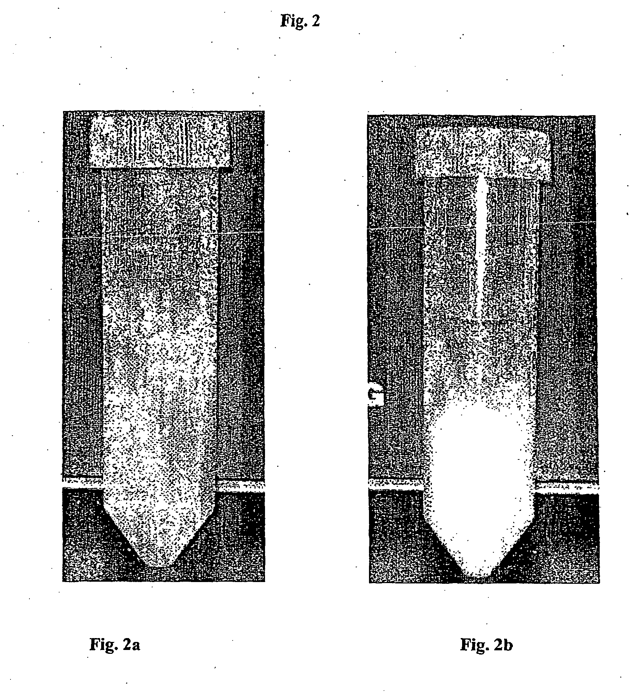

The phase stability of liposome preparations of the present invention affords pharmaceutically useful liposomes. The phase stability of the preparations as noted in **Example 1** were monitored visually for the formation of sediment or aggregates. Liposome compositions that are not sufficiently phase stable, typically show sedimentation very rapidly, often overnight, while others show no signs of sedimentation even after years of storage. An example of the visual appearance of phase stable liposomes is shown in **Figure 2a** whilst an example of the visual appearance of a phase unstable preparation in **Figure 2b** shows obvious sedimentation and phase separation.

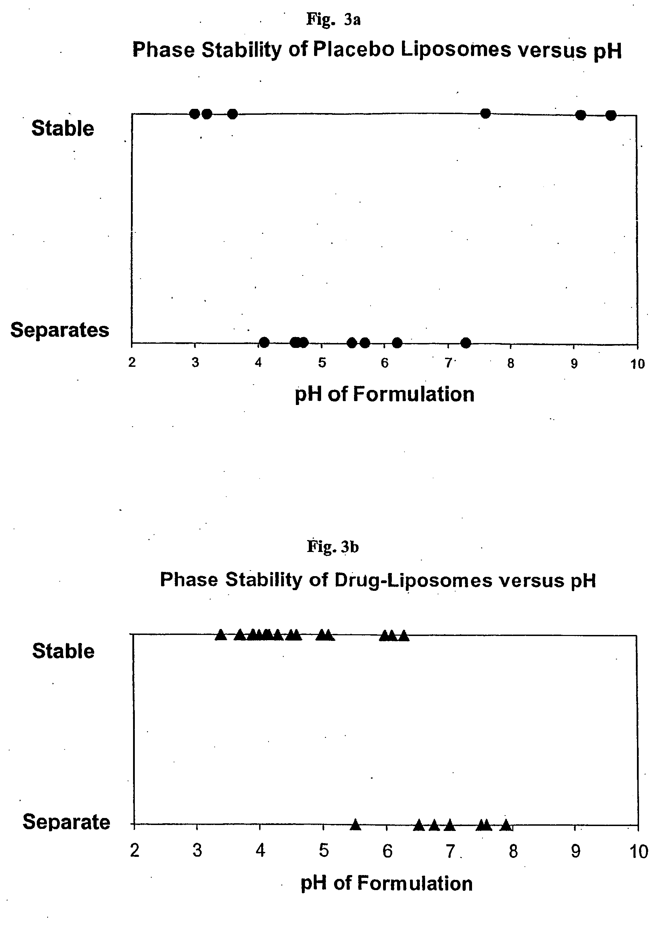

Referring to **Figure 1**, placebo liposome compositions lacking pharmaceutical agent, are generally phase unstable and rapidly sedimented. Over the pH range of 4 to 7, placebo liposomes of Figure 1 that have not been autoclaved are phase unstable (**Figure 3a**). Placebo liposome preparations that appeared to be phase stable were generally associated with the extremes of pH, namely less than pH 3 and greater than pH 8, where chemical stability is generally compromised.

Referring again to **Figure 1**, the addition of the salt of a lipophilic amine was found to have a stabilizing effect on the liposome preparation. In contrast, a liposome preparation containing only the lipophilic amine fentanyl (without an acid counter-ion) is unstable. However, as the ratio of acid in the formulation increased, the phase stability of the preparation increased. The stabilizing effect of the lipophilic amine in combination with the appropriate concentration of acid, provides liposome preparations that are phase stable at pH values less than about 7 as shown in **Figure 3b** prior to autoclaving. In contrast to the placebo formulations the liposome preparations containing a lipophilic amine are phase stable over the pH range of about pH 4 to about pH 6 with the exception of preparations that contain sodium chloride.

### Example 3 - Stability of various liposome encapsulated drug compositions, including autoclaved compositions.

In the pharmaceutical industry, sterile formulations can be "terminally sterilized", i.e., they are autoclave sterilized after being filled into individual vials. End-sterilizing liposome encapsulated drug products of the present invention via autoclaving can provide individually packaged stable and sterile liposome compositions suitable for use in the pharmaceutical industry.

As set out above, various prior art references relating to the autoclaving of liposomes evidence the widely held view by skilled persons in the art that liposomes, in particular those based on phosphatidylcholine, are fragile and unstable to the harsh conditions of autoclaving leading to agglomeration of liposomes, change in liposome size or size distribution, hydrolysis/oxidation of lipids, chemical degradation and undesired release of the encapsulated drug (for example, see WO 2004/002468). As described herein, embodiments of the liposome compositions of the present invention can not only withstand autoclaving, but also appear to have enhanced phase stability following autoclaving. Moreover, the following parameters relating to the stability of the liposome compositions of the present invention were measured both before and after autoclaving to further evidence the stabilizing effect of autoclaving on compositions of the present invention: pH, particle size distribution, lipid hydrolysis, lipid oxidation, and phase stability. A discussion on each of these parameters follows.

Liposome preparations were prepared with various lipophilic amines added as the base in combination with varying amounts of different acids. Each test batch of liposome preparation was prepared with 1.2 g purified soya lecithin, 0.12 g of cholesterol, and a the base of the lipophilic amine dissolved in 3 gm of ethanol and warmed to 56 degrees Celsius. An aqueous phase of 27 gm water for injection optionally contained various acids or salts was warmed to 56 degrees Celsius. In formulations where the test acid was palmitic acid, the acid was dissolved in the ethanolic phase while all other acids were dissolved in the aqueous phase. Following dissolution of all constituents in the ethanolic phase, the ethanolic solution was added the aqueous solution, the mixture was shaken at 56 degrees Celsius for 10 minutes on an orbital shaker and then gradually cooled to ambient temperature. The presence of multilamellar liposomes was confirmed by microscopy. Samples of each liposome preparation were transferred to sealed glass vials and autoclaved at 121 degrees Celsius for 20 minutes.

A variety of parameters of the liposome compositions were measured both before and after autoclaving to characterize both physical stability and chemical stability of the compositions.

**Figure 4** provides a summary of the liposome preparations, the pH, the particle size, before and after autoclaving, and the phase stability index, before and after autoclaving.

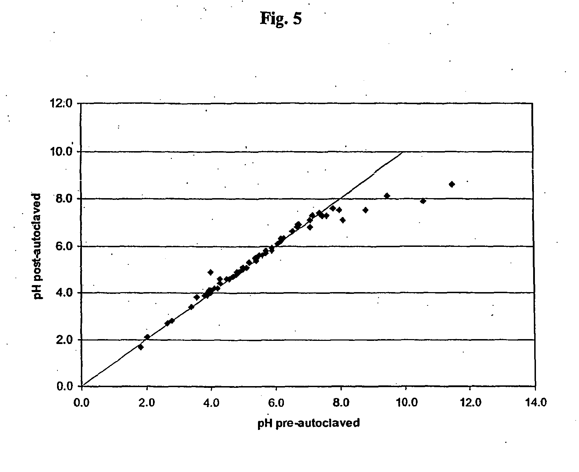

**Stability with respect to pH:** A concern with autoclaving liposomes is the potential for chemical degradation of phospholipid or drug components. Chemical degradation in a formulation is often reflected by changes in pH. As shown in **Figure 5,** the relationship between pH after autoclaving versus pH before autoclaving liposome compositions of the present invention can be generally described by the equation pH(post) = pH(prior), indicating no change in pH for formulations having an initial pH of less than 7. Liposome preparations that were slightly alkaline prior to autoclaving, specifically formulations with pH > 8, had lower pH after autoclaving and the points on the graph deviated from the desired linear relationship. Autoclaving formulations at higher pH values appears to result in chemical degradation of components of the formulation.

**Impact of Autoclaving on Phosphatidylcholine Content:** A concern with autoclaving liposomes is the potential for hydrolysis of ester based phospholipids such as phosphatidylcholine. The phosphatidylcholine concentrations of twelve formulations of the present invention before and after autoclaving were determined. The phosphatidylcholine concentrations of twelve differing liposomal formulations before and after autoclaving were determined. Phosphatidyl choline and its related hydrolysis product lysophosphatidyl choline were tested by normal phase HPLC using a silica-diol column with Evaporative Light scattering detection, and a gradient from eluent A (n-hexane: 2-propanol: acetic acid: triethylamine 81.4:17:1.5:0.8) to eluent B (2-propanol:water:acetic acid: triethylamine 84.4:14:1.5:0.08)over 15 minutes at 1.5 to 2 mL/min flow rate.

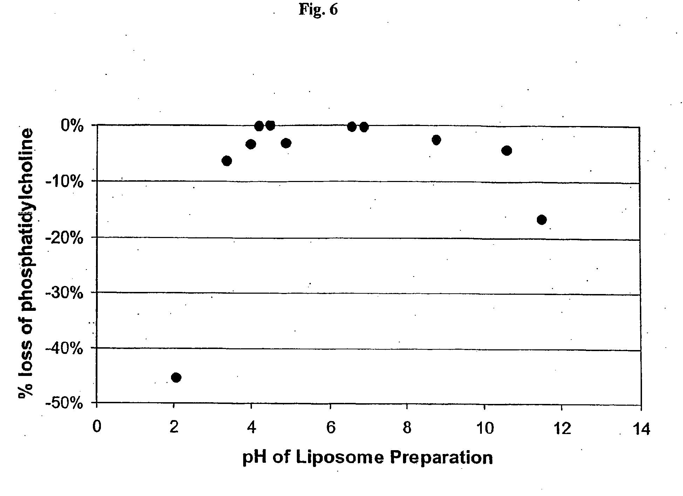

Twelve formulations from Figure 4 were selected to covered the full range pH values of the formulations of **Figure 4.** Although hydrolysis can be significant at pH values less than 4 or pH values greater than 10 as shown in Figure 6, liposome preparations of the present invention between 4 and 9, and more preferably between pH 4 and pH 7, could be autoclaved without significant loss of phospholipid, typically less than 10% loss, more preferably less than 5% loss.

**Impact of Autoclaving on Lipid Oxidation:** Oxidation of lipids during autoclaving was minimized by preparing and filling formulations under an inert atmosphere. **Table 2** below summarizes the changes in lipid oxidation after autoclaving for a liposomal compositions containing as a pharmaceutical agent, fentanyl:citric acid (1:1). The oxidation of lipid components was tested by a colorimetric assay. All samples containing lipids, and standards, were reacted with ferrous oxidation/xylenol orange (FOX) reagent and measured at 562 nm. Blanks were prepared by reaction with triphenylphosphine, measured at 562nm and subtracted from the absorbance reading of samples. Quantitation was performed versus a standard curve of cumene hydroperoxide. Exclusion of oxygen was observed to prevent oxidation during the autoclaving process.

**Table 2**

| Filling conditions | % Increase in oxidized lipid content after autoclaving |
|---|---|
| Argon | 0% |
| Nitrogen | 0% |
| Oxygen | 340% |
| Air | 155% |

Impact of Autoclaving on the Phase Stability Index: The phase stability of liposome compositions of the present invention affords pharmaceutically useful liposome compositions. Liposome compositions that are not sufficiently phase stable typically show sedimentation of the type shown in Figure 2b, very rapidly, often overnight, while others can sediment only after weeks of storage. The desired shelf life for a pharmaceutical product in some embodiments is two years or more under specified storage condition. For example, in one embodiment liposome compositions of the present invention containing fentanyl citrate as the active ingredient/organic acid have shown that the product retains phase stability for over two years at 4 degrees Celsius.

During formulation development for new drug candidates, it is impractical to monitor stability in real time of a period of months or years to evaluate phase stability. As a result, we have developed a method using centrifugation to provide an analytical tool for predicting the long-term phase stability of a liposome composition.

In the assay, liposomal compositions were prepared as described herein and summarized in Figure 4. Particle size distribution of the liposomes is conducted by a light scattering method employing a Malvern Mastersizer 2000, after dilution of the liposomes in dispersant in water for injection. A sample of the liposomal preparations was withdrawn and the particle size distribution was measured using a Malvern Mastersizer. The mass median diameter (d(0.5)) of the liposome composition was recorded. A 3 ml aliquot of each liposome preaparton was centrifuged at a 2,292g and 4°C for 2 hours. Following centrifugation, an aliquot of the sample was withdrawn from the top layer of the centrifuged sample and the particle size distribution measured using the Malvern Mastesizer. The mean mass diameter of the top layer of supernatant was recorded.

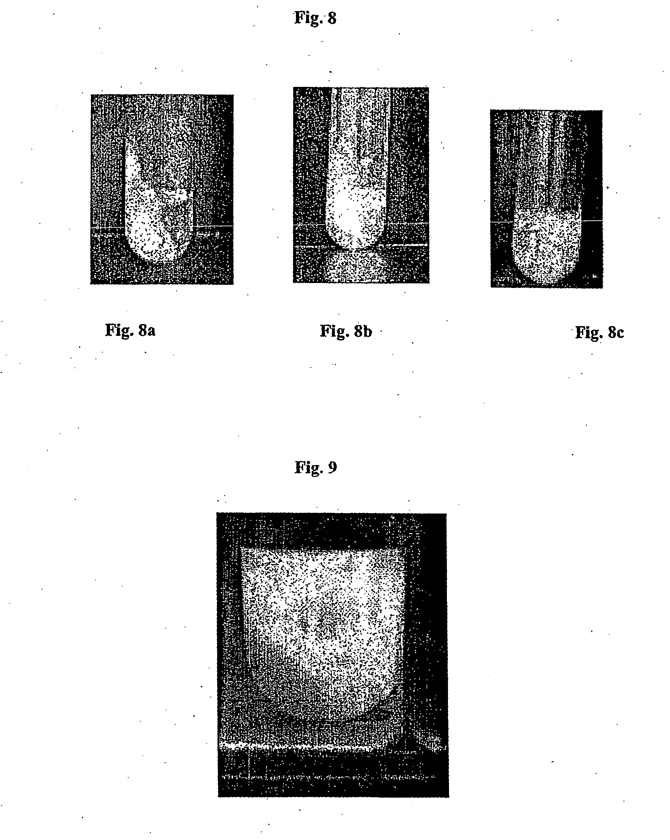

An example of liposome compositions tested by the present protocol is shown in **Figures 8a-8c.** When the sample was phase unstable, the centrifugation provided a solid pellet at the bottom of the centrifuge tube, and a clear supernatant, **Figure 8a.** Upon measurement with the Malvern Mastersizer, essentially no particles were detected in the supernatant as confirmed by an obscuration value of essentially zero. Obscuration is well known by persons skilled in the art as being a suitable test for determining the volume amount of liposomes. When the sample was phase stable, no pellet was visible after centrifugation and the sample in the centrifuge tube remained a homogeneous dispersion. Upon measurement with the Malvern Mastersizer, the obscuration value confirmed the presence of numerous liposomes in the supernatant and the mass median diameter of the liposomes was generally 60% - 100% of the value recorded in the starting formulation.

Using the d(0.5) value as the marker for the consistency of particle size distribution, we define a Phase Stability Index (PS Index) by the following equation

$$\text{Phase Stability Index} = d(0.5) \text{ post-centrifugation}/d(0.5) \text{ pre-centrifugation}$$

When the sample was phase stable as defined herein, following centrifugation there was no visible pellet and the liposomes appeared to remain uniformly distributed throughout the liquid phase **Figure 8c**, and the PS Index was about greater than about 0.6.

If the sample had intermediate phase stability, following centrifugation a density gradient or partial pellet were noted on visual inspection as in **Figure 8b**, and typically, a PS Index of greater than or equal to 0.1 but about less than or equal 0.6. To further illustrate compositions with intermediate phase stability, **Figure 9** shows a photograph of a liposomal composition of intermediate phase stability. After centrifugation, some of the liposomes have settled and a pellet is visible. The supernatant was not clear, with some liposomes remaining in suspension. This picture was taken with backlighting to permit visualization of the demarcation between the pellet and the supernatant.

The PS Index for various liposome compositions was measured using this method both prior to and after autoclaving the compositions, and the data are summarized in **Figure 4.**

Overall, formulations containing only the free base of the drugs were generally phase unstable before and after autoclaving. The only exception was the formulation with Sumatriptan. However, Sumatriptan carries a charge at the pH of the final formulation whereas all the other molecules are uncharged or only partially charged at the pH of the final formulation.

Overall, the autoclaving of the liposome formulations increased the number of liposome formulations that were characterized by a PS Index greater than 0.6 as summarized in Table 3 as follows:

**Table 3**

| PS Index | % of Formulations Pre-Autoclave | % Formulations Post-Autoclave |
|---|---|---|
| PS Index <0.1 | 51% | 25% |
| 0.1≤ PS Index ≤0.6 | 23% | 13% |
| PS Index > 0.6 | 26% | 62% |

Prior to autoclaving, 51% of the formulations generally had poor or no phase stability, with only 26% of the formulations having excellent phase stability. Following autoclaving, the number of phase stable liposome preparations increased dramatically to over 62% of all the preparations.

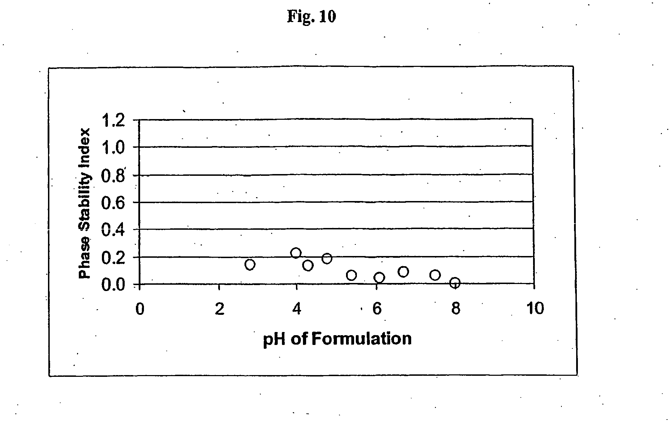

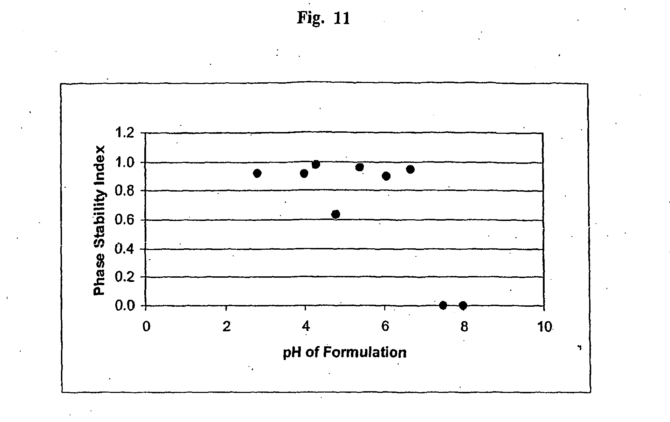

With the addition of the appropriate amount of an acid, it was possible to titrate the pH of the preparations to ranges below the pKa such that an increasing amount of the amino group of the lipophlic amine drug was protonated. At pH ranges between about 4 and about 7, and following the autoclaving step, the liposomes displayed remarkable phase stability. **Figure 10** and **Figure 11** compare the pre and post-autoclaved Phase Stability Index of liposome preparations containing fentanyl as the lipophilic amine of **Figure 4.** In **Figure 10,** the graphs show that prior to autoclaving the formulations were prone to sedimentation during centrifugation, as reflected by a low PS Index. In contrast, after autoclaving, all formulations with pH values below that of the $pK_a$ of fentanyl ($pK_a$ = 7.3) were phase stable.

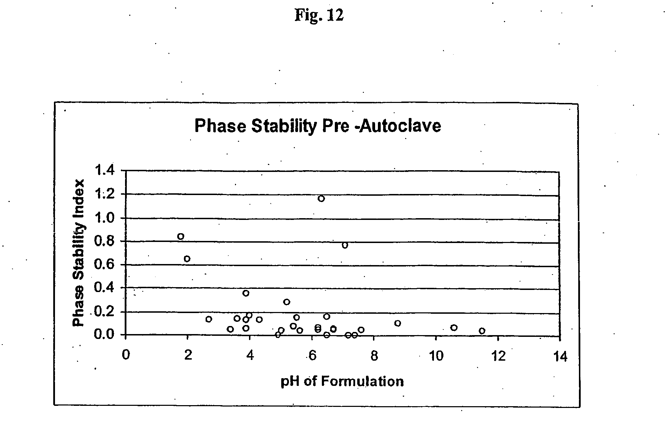

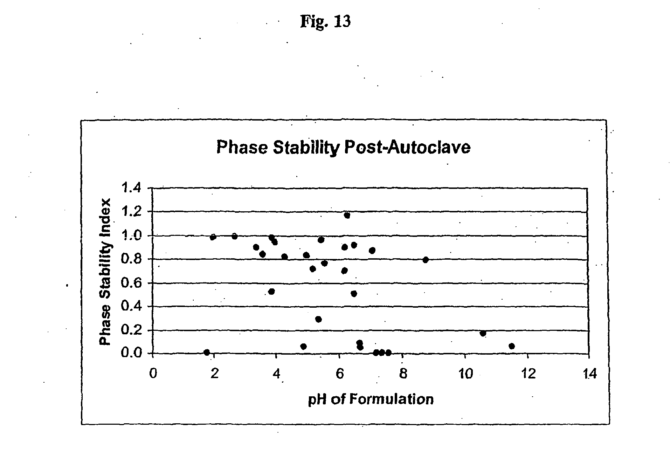

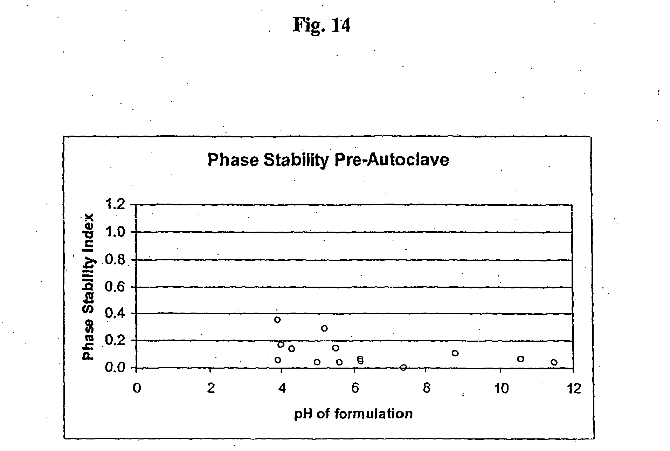

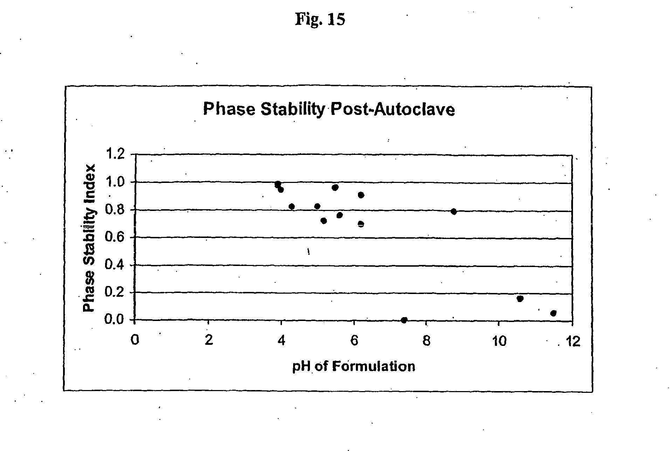

Referring to **Figure 12** and **Figure 13,** these graphs compare the pre- and post-autoclaved Phase Stability Index of liposome preparations of **Figure 4** containing ondansetron ($pK_a$ - 7.4) as the lipophilic amine. For the compositions with ondansetron, there was more scatter observed in the data than for fentanyl, and this appears to stem from two main classes of formulations, those containing palmitic acid and those containing high ondansetron concentrations. The palmitic acid containing formulations behaved less well as they tended to form viscous mixtures and the liposome content was less uniform. At high ondansetron concentration (2.4 mM) visible crystals of drug were observed. When these two subclasses of formulations are removed, the trend for the ondansetron formulations is consistent with fentanyl, namely that after autoclaving, formulations with pH values below the $pK_a$ of the lipophilic amine are phase stable, **Figures 14** and **15**.

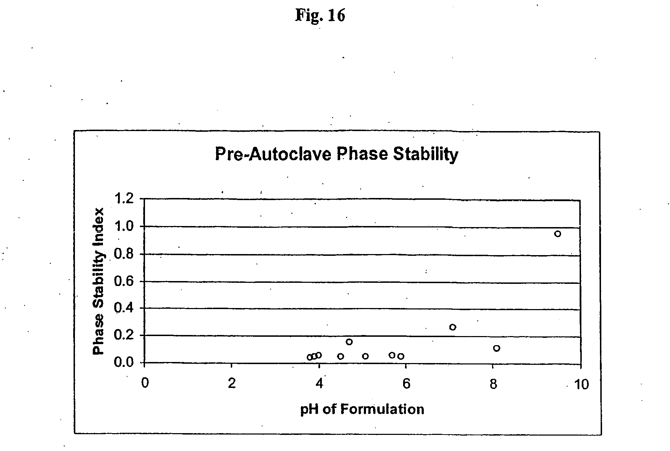

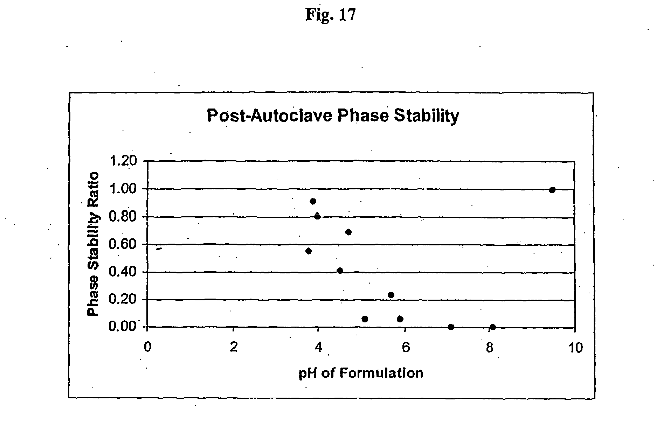

Referring to **Figure 16** and **Figure 17,** relating to compositions containing sumatriptan of **Figure 4**, only the formulation with the free drug was phase stable upon initial preparation. As with other drugs, titrating the formulation to lower pH imparts phase stability after autoclaving to a larger number of formulations. The required pH is lower for sumatriptan. Importantly, while sumatriptan is a lipophilic amine, it also has a sulfonamide group in its structure, has multiple ionisable groups and corresponding $pK_a$ values and has the lowest octanol water partition coefficient (Log P = 1.05)

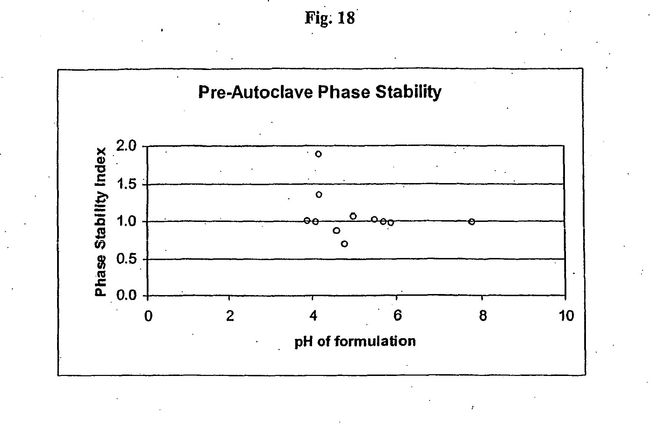

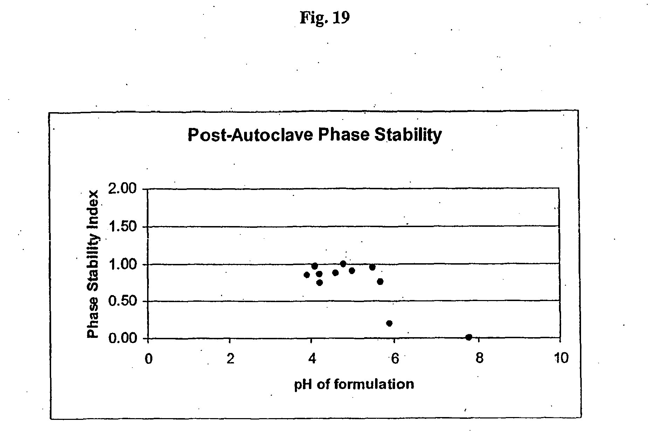

Referring to **Figure 18** and **Figure 19**, relating to compositions containing prochlorperazine of **Figure 4**, the formulations were found to be phase stable both prior to as well as after autoclaving. The autoclaving was shown to have a negative impact only on formulations at higher pH values, not unlike formulations with other drugs. Prochlorperazine has a higher $pK_a$ of 8.1which is higher than either ondansetron or fentanyl. Many of the formulations prepared with ondansetron were fully 2 pH units lower than the pKa.

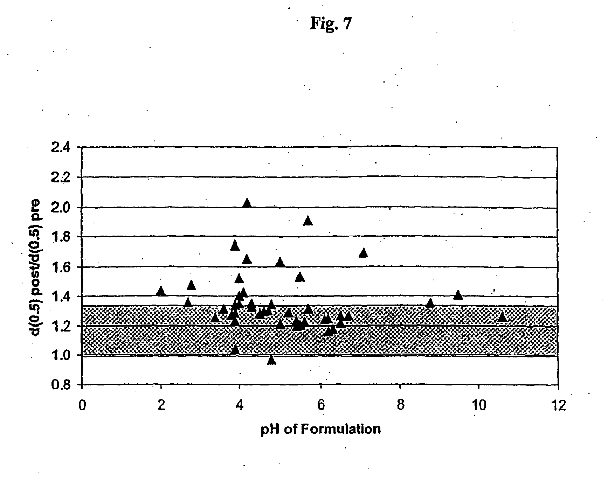

**Particle Size of Liposomes:** Prior art suggested that liposomes should change significantly in size following autoclaving. Particle size distribution of the liposomes is conducted by a light scattering method employing a Malvern Mastersizer 2000, after dilution of the liposomes in dispersant in water for injection. The results from our studies using preparations with PS Index > 0.6 after autoclaving, show that the measured d(0.5) of the liposomes, measured prior to centrifugation of the native preparation or the autoclaved preparation, increases fractionally after the autoclaving with most of the formulations showing less than a 33% increase in liposome size. Greater changes in liposome size were recorded for formulations with pH values below pH 4 or above pH 7 as shown in Figure 7. As discussed above, the preferred pH range for the formulations is about between 4-7, and preferably between 5-6, to optimize the physical and chemical stability of the entire formulation.

## Example 4 - Storage Stability

Preparations containing a mixture of free fentanyl and liposome encapsulated fentanyl were prepared by mixing an ethanolic phase with an aqueous phase at various batch sizes. The ethanolic phase comprised ethanol, fentanyl citrate, phosphatidylcholine and cholesterol. The aqueous phase comprised water for injection. Before mixing, both phases were heated to a temperature of about 56 to 60 degrees centigrade. The two phases were mixed and the mixture was stirred for a further 10 minutes at 56-60 degrees centigrade. The mixture was then allowed to cool to room temperature over approximately two hours. Typically, each ml of the final aqueous formulation contained 500 mcg fentanyl (as 800 mcg of fentanyl citrate), 40 mg phosphatidylcholine, 4 mg cholesterol, and 100 mg ethanol, in a solution of water for injection. After filling, preparations were autoclaved for final sterilization (some air was present). Final preparations contained between 30 to 40% of the fentanyl as free drug with the remainder (70 to 60%) in the encapsulated fraction.

**Table 4**

| Lot Size | End Storage Time at 4°C | Particle Size d (0.5) (microns) | | Percent Encapsulated | | Phosphatidyl-Choline (mg/ml) | |
|---|---|---|---|---|---|---|---|
| | | T = 0 | End time | T =0 | End time | T=0 | End time |
| 1 litre | 13 months | 5.7 | 5.7 | - | - | - | - |
| 2.5 litres | 20 months | 6.4 | 6.3 | 66.6 | 64.9 | 33.5 | 32.8 |
| 15 litre | 12 months | 6.1 | 6.3 | 63.9 | 65.5 | 33.6 | 34.8 |

The aqueous liposomal preparations were stored at 4 degrees Celsius and monitored for stability of particle size distribution and changes in drug encapsulation. Particle size distribution of the liposomes is conducted by a light scattering method employing a Malvern Mastersizer 2000, after dilution of the liposomes in dispersant, filtered Water for Irrigation. The percent encapsulation of the drug within the liposomes is tested by centrifuging the liposomes at high centrifugal force (gmax 277816), at 4oC for two hours. The extreme conditions were necessary to create a proper pellet for the very phase stable formulations. The supernatant and pellet were both analyzed for drug by reverse phase HPLC using a C8 column with UV detection and buffer of 40/40/20 ammonium acetate buffer/methanol/acetonitrile. The neat sample was also injected, to calculate mass balance. Percent encapsulated is calculated by:

$$\%E = \frac{C(pellet)}{C(pellet) + C(supernatant)} \times 100\%$$

wherein C(pellet) is the concentration of drug in the pellet and C(supernatant) is the concentration of drug in the supernatant.

No significant changes in particle size distribution, percent encapsulation or phosphatidylcholine content were observed. The preparations were phase stable by visual inspection and for samples from the 15 litre lot the Particle Size Index was 0.72 after 19 months storage.

Although embodiments of the invention have been described herein, it will be understood by those skilled in the art that variations may be made thereto without departing from the spirit of the invention or the scope of the appended claims. It will be further understood by those skilled in the art that elements of the different embodiments of the present invention maybe combined in any logical manner.

**Claims**

1.  A method of increasing the stability of liposome compositions, said method comprising the steps of:

(a) providing a suitable aqueous medium;
(b) providing a suitable phospholipid;
(c) providing at least one pharmaceutical agent being capable of being at least partially encapsulated in the liposomes, and being selected from:

(i) a lipophilic amine and a pharmaceutically acceptable acid, wherein the pharmaceutically acceptable acid is selected from an organic or inorganic acid, and
(ii) a pharmaceutically acceptable organic acid salt of a lipophilic amine, and optionally a pharmaceutically acceptable organic acid;

wherein quantity of the pharmaceutically acceptable acid present in the composition is such that the pH of the liposome composition is less than or approximately equal to the $pK_a$ of the amino group of the pharmaceutically active lipophilic amine;

(d) combining the aqueous medium, phospholipid and pharmaceutical agent to form the liposome composition; and

(e) autoclaving said liposome composition at conditions effective to sterilize said compositions, thereby affording compositions with increased stability relative to the stability of the composition prior to autoclaving.

2. The method according to 1, wherein the step of autoclaving is carried out at a temperature of about 121°C for a minimum of about 15 minutes under an inert atmosphere.

3. The method of claim 2, wherein the inert atmosphere during autoclaving comprises argon or nitrogen.

4. A method of identifying a phase stable liposome composition, the method comprising the steps of:

(a) providing a liposome composition comprising a phospholipid, an aqueous solution, a pharmaceutical agent, and optionally ethanol and a sterol,
(b) optically determining the mass median diameter (d(0.5)) value of the liposome composition;
(c) centrifuging the liposome composition at between about 1000g and about 5000g, at about 4°C for about 2 hours;
(d) optically determining the mass median diameter (d(0.5)) value of the supernatant portion of the liposome composition solution after centrifugation step (c);
(e) calculating the ratio of the mass median diameter (d(0.5)) particle size distribution value of the solution after centrifugation to that of the solution prior to centrifugation;

wherein a phase stable liposome composition is identified as such if the composition has a ratio in step (e) of about 0.6 or greater.

5. A method of claim 4, wherein the phase stable liposome composition is identified as such if the composition has a ratio in step (e) of about 0.8 or greater.

6. A method of claim 4, wherein prior to centrifugation the composition is autoclaved.

7. A liposome composition having a stability that has been increased by the method of any one of claims 1-3.

8. A phase stable liposome composition identified by the method of any one of claims 4-6.

## Fig. 1.

| Formulation | Drug | [Drug] mM | Acid | [Acid] mM | Salt | [Salt] mM | pH | Phase Stability Pre Autoclave | Phase Stability Post Autoclave |
|---|---|---|---|---|---|---|---|---|---|
| P01 | - | - | - | - | - | - | 4.6 | separates | |
| P02 | - | - | citric | 0.1 | - | - | 4.1 | separates | |
| P03 | - | - | citric | 0.5 | - | - | 3.6 | stable | separates |
| P04 | - | - | maleic | 1.5 | - | - | 3.0 | stable | stable |
| P04 | - | - | phosphoric | 0.1 | - | - | 4.1 | separates | |
| P06 | - | - | phosphoric | 0.5 | - | - | 3.6 | stable | |
| P07 | - | - | citric | 1.5 | - | - | 3.2 | stable | separates |
| P08 | - | - | | - | monosodium malate | 1.5 | 4.6 | separates | |
| P09 | - | - | | - | potassium phosphate | 1.5 | 4.7 | separates | |
| P10 | - | - | | - | potassium tartrate | 1.5 | 5.7 | separates | |
| P11 | - | - | | - | sodium acetate | 1.5 | 6.2 | separates | |
| P12 | - | - | | - | sodium bicarbonate | 1.5 | 7.6 | stable | |
| P13 | - | - | | - | sodium chloride | 150 | 5.5 | separates | |
| P14 | - | - | | - | sodium citrate | 1.5 | 7.3 | separates | |
| P15 | - | - | | - | sodium phosphate | 1.5 | 9.6 | stable | |
| P16 | - | - | | - | sodium pyrophosphate | 1.5 | 9.2 | stable | |
| P17 | - | - | | - | sodium tartrate | 1.5 | 5.7 | separates | |
| P18 | Fentanyl | 1.5 | - | - | - | - | 7.9 | separates | separates |
| P19 | Fentanyl | 1.5 | citric | 0.1 | - | - | 7.5 | separates | separates |
| P20 | Fentanyl | 1.5 | citric | 0.5 | - | - | 6.5 | separates | separates |
| P21 | Fentanyl | 1.5 | citric | 1.5 | - | - | 4.2 | stable | stable |
| P22 | Fentanyl | 3 | citric | 3 | - | - | 4.1 | stable | stable |
| P23 | Fentanyl | 4.5 | citric | 4.5 | - | - | 4.0 | stable | stable |
| P24 | Fentanyl | 1.5 | citric | 1 | - | - | 4.6 | stable | stable |
| P25 | Fentanyl | 1.5 | citric | 2 | - | - | 3.7 | stable | stable |
| P26 | Ondansetron | 1.5 | hydrochloric | 1.5 | - | - | 4.3 | stable | |
| P27 | Fentanyl | 1.5 | maleic | 0.1 | - | - | 7.6 | separates | separates |
| P28 | Fentanyl | 1.5 | maleic | 0.5 | - | - | 7.0 | separates | separates |
| P29 | Fentanyl | 1.5 | maleic | 1 | - | - | 6.3 | stable | stable |
| P30 | Fentanyl | 1.5 | maleic | 1.5 | - | - | 5.1 | stable | stable |
| P31 | Fentanyl | 1.5 | maleic | 2 | - | - | 3.4 | stable | stable |
| P32 | Prochlorperazine | 1.5 | maleic | 3 | - | - | 3.4 | stable | |
| P33 | Dihydroergotamine | 1.5 | methanesulfonic | 1.5 | - | - | 4.0 | stable | |
| P34 | Doxylamine | 1.5 | succinic | 1.5 | - | - | 5.0 | stable | |
| P35 | Sumatriplan | 1.5 | succinic | 1.5 | - | - | 5.0 | stable | |
| P36 | Fentanyl | 1.5 | citric | 1.5 | monosodium malate | 1.5 | 4.1 | stable | |
| P37 | Fentanyl | 1.5 | citric | 1.5 | potassium tartrate | 1.5 | 4.6 | stable | |
| P38 | Fentanyl | 1.5 | citric | 1.5 | sodium acetate | 1.5 | 6.0 | stable | |
| P39 | Fentanyl | 1.5 | citric | 1.5 | sodium acetate | 75 | 6.1 | stable | |
| P40 | Fentanyl | 1.5 | citric | 1.5 | sodium chloride | 75 | 6.8 | separates | |
| P41 | Fentanyl | 1.5 | citric | 1.5 | sodium chloride | 150 | 5.5 | separates | |
| P42 | Fentanyl | 1.5 | citric | 1.5 | sodium citrate | 1.5 | 3.9 | stable | |
| P43 | Fentanyl | 1.5 | citric | 1.5 | sodium citrate | 75 | 3.9 | stable | |
| P44 | Fentanyl | 1.5 | citric | 1.5 | sodium tartrate | 1.5 | 4.5 | stable | |

Fig. 2

Fig. 2a

Fig. 2b

Fig. 3a

## Phase Stability of Placebo Liposomes versus pH

Fig. 3b

## Phase Stability of Drug-Liposomes versus pH

## Fig. 4

| ID Number | Drug | [Drug] mM | Acid | [Acid] mM | pH | d(0.5) (microns) Pre-autoclave | d(0.5) (microns) Post autoclave | Phase Stability Index Pre Autoclave | Phase Stability Index Post Autoclave |
|---|---|---|---|---|---|---|---|---|---|
| B01 | Fentanyl | 1.5 | - | - | 8.00 | 3.18 | 4.59 | 0.00 | 0.00 |
| B02 | Ondansetron | 1.5 | - | - | 7.40 | 3.21 | 0.00 | 0.00 | 0.00 |
| B03 | Ondansetron | 2.4 | - | - | 7.60 | 3.24 | 4.32 | 0.05 | 0.00 |
| B04 | Sumatriptan | 1.5 | - | - | 9.50 | 3.52 | 4.95 | 0.95 | 0.99 |
| B05 | Prochlorperazine | 1.5 | - | - | 7.80 | 9.18 | 0.00 | 0.99 | 0.00 |
| B06 | Ondansetron | 2.4 | Palmitic | 0.2 | 7.20 | 10.31 | 10.14 | 0.00 | 0.00 |
| B07 | Ondansetron | 2.4 | Succinic | 2.4 | 4.90 | 3.98 | 4.65 | 0.00 | 0.05 |
| B08 | Ondansetron | 2.4 | Succinic | 0.2 | 6.50 | 3.77 | 4.60 | 0.00 | 0.50 |
| B09 | Ondansetron | 1.5 | Succinic | 0.75 | 5.60 | 3.89 | 4.77 | 0.04 | 0.76 |
| B10 | Ondansetron | 1.5 | Sodium Phosphate | 15.0 | 11.50 | 6.09 | 6.10 | 0.04 | 0.06 |
| B11 | Fentanyl | 1.5 | Acetic | 0.75 | 6.10 | 3.93 | 4.89 | 0.04 | 0.89 |
| B12 | Ondansetron | 1.5 | Succinic | 1.5 | 5.00 | 3.85 | 4.66 | 0.04 | 0.83 |
| B13 | Ondansetron | 2.4 | Succinic | 24.0 | 3.40 | 4.49 | 5.62 | 0.04 | 0.90 |
| B14 | Sumatriptan | 1.5 | Phosphoric | 1.5 | 3.80 | 3.84 | 4.88 | 0.04 | 0.55 |
| B15 | Ondansetron | 2.4 | Maleic | 0.2 | 6.70 | 3.60 | 4.65 | 0.04 | 0.05 |
| B16 | Sumatriptan | 1.5 | Citric | 1.0 | 4.50 | 3.56 | 4.55 | 0.04 | 0.41 |
| B17 | Sumatriptan | 1.5 | Maleic | 1.0 | 5.90 | 3.47 | 4.88 | 0.05 | 0.06 |
| B18 | Sumatriptan | 1.5 | Citric | 0.75 | 5.10 | 3.19 | 4.23 | 0.05 | 0.05 |
| B19 | Sumatriptan | 1.5 | Maleic | 1.5 | 3.90 | 3.92 | 5.25 | 0.05 | 0.91 |
| B20 | Ondansetron | 1.5 | Phosphoric | 0.75 | 6.20 | 3.53 | 4.10 | 0.05 | 0.70 |
| B21 | Fentanyl | 1.5 | Citric | 0.2 | 7.50 | 3.41 | 4.36 | 0.06 | 0.00 |
| B22 | Ondansetron | 2.4 | Citric | 0.2 | 6.70 | 3.33 | 4.25 | 0.06 | 0.08 |
| B23 | Sumatriptan | 1.5 | Citric | 1.5 | 4.00 | 3.33 | 5.07 | 0.06 | 0.80 |
| B24 | Sumatriptam | 1.5 | Succinic | 0.75 | 5.70 | 3.50 | 4.60 | 0.06 | 0.23 |
| B25 | Ondansetron | 1.5 | Phosphoric | 1.5 | 3.90 | 3.55 | 4.37 | 0.06 | 0.98 |
| B26 | Fentanyl | 1.5 | Acetic | 1.5 | 6.40 | 3.86 | 4.75 | 0.06 | 0.96 |
| B27 | Ondansetron | 1.5 | Sodium Phosphate | 1.5 | 10.60 | 4.05 | 5.11 | 0.06 | 0.16 |
| B28 | Ondansetron | 1.5 | Maleic | 0.75 | 6.20 | 3.84 | 4.80 | 0.07 | 0.90 |
| B29 | Ondansetron | 2.4 | Maleic | 2.4 | 5.40 | 3.80 | 4.55 | 0.08 | 0.29 |
| B30 | Fentanyl | 1.5 | Phosphoric | 0.75 | 6.70 | 3.56 | 4.51 | 0.08 | 0.94 |
| B31 | Ondansetron | 1.5 | Sodium Phosphate | 0.2 | 8.80 | 3.56 | 4.82 | 0.10 | 0.80 |
| B32 | Sumatriptan | 1.5 | Maleic | 0.75 | 8.10 | 3.15 | 0.00 | 0.11 | 0.00 |
| B33 | Ondansetron | 2.4 | Citric | 24.0 | 2.70 | 4.33 | 5.87 | 0.13 | 0.99 |
| B34 | Ondansetron | 1.5 | Citric | 1.0 | 4.30 | 3.48 | 4.61 | 0.13 | 0.82 |
| B35 | Ondansetron | 2.4 | Citric | 2.4 | 3.90 | 3.59 | 4.61 | 0.13 | 0.52 |
| B36 | Fentanyl | 1.5 | Phosphoric | 1.5 | 4.30 | 3.42 | 4.62 | 0.13 | 0.97 |
| B37 | Fentanyl | 1.5 | Citric | 15.0 | 2.80 | 4.22 | 6.22 | 0.14 | 0.92 |
| B38 | Ondansetron | 2.4 | HCl | 2.4 | 3.60 | 3.58 | 4.70 | 0.15 | 0.84 |
| B39 | Ondansetron | 1.5 | Maleic | 1.0 | 5.50 | 3.84 | 4.64 | 0.15 | 0.96 |
| B40 | Sumatriptam | 1.5 | Succinic | 1.5 | 4.70 | 3.84 | 4.99 | 0.15 | 0.69 |
| B41 | Ondansetron | 2.4 | HCl | 0.2 | 6.50 | 3.57 | 4.52 | 0.16 | 0.92 |
| B47 | Ondansetron | 1.5 | Citric | 1.5 | 4.00 | 3.67 | 4.95 | 0.17 | 0.94 |
| B48 | Fentanyl | 1.5 | Citric | 1.0 | 4.80 | 3.48 | 4.68 | 0.18 | 0.63 |
| B49 | Fentanyl | 1.5 | Citric | 1.5 | 4.00 | 3.60 | 5.05 | 0.22 | 0.91 |
| B50 | Sumatriptan | 1.5 | Phosphoric | 0.75 | 7.10 | 3.39 | 0.00 | 0.26 | 0.00 |
| B60 | Ondansetron | 1.5 | Citric | 0.75 | 5.20 | 3.23 | 4.17 | 0.29 | 0.72 |
| B61 | Ondansetron | 1.5 | Maleic | 1.5 | 3.90 | 3.60 | 3.73 | 0.36 | 0.98 |
| B62 | Ondansetron | 2.4 | Maleic | 24.0 | 2.00 | 5.00 | 7.19 | 0.65 | 0.98 |
| B63 | Prochlorperazine | 1.5 | Citric | 0.75 | 4.80 | 5.37 | 5.19 | 0.68 | 0.99 |
| B64 | Ondansetron | 2.4 | Palmitic | 2.4 | 7.10 | 3.36 | 5.70 | 0.77 | 0.87 |
| B65 | Ondansetron | 2.4 | HCl | 24.0 | 1.80 | 5.47 | 0.00 | 0.83 | 0.00 |
| B66 | Prochlorperazine | 1.5 | Succinic | 1.5 | 4.60 | 4.77 | 6.17 | 0.86 | 0.87 |
| B67 | Prochlorperazine | 1.5 | Maleic | 0.75 | 5.90 | 3.44 | 28.70 | 0.96 | 0.20 |
| B68 | Prochlorperazine | 1.5 | Citric | 1.0 | 4.10 | 4.19 | 5.97 | 0.98 | 0.96 |
| B69 | Prochlorperazine | 1.5 | Phosphoric | 0.75 | 5.70 | 3.51 | 6.71 | 0.98 | 0.76 |
| B70 | Prochlorperazine | 1.5 | Citric | 1.5 | 3.90 | 3.84 | 6.70 | 1.01 | 0.85 |
| B71 | Prochlorperazine | 1.5 | Succinic | 0.75 | 5.50 | 4.01 | 6.16 | 1.02 | 0.95 |
| B72 | Prochlorperazine | 1.5 | Maleic | 1.0 | 5.00 | 3.24 | 5.30 | 1.05 | 0.91 |
| B73 | Ondansetron | 2.4 | Palmitic | 24.0 | 6.30 | 8.71 | 10.26 | 1.16 | 1.17 |
| B74 | Prochlorperazine | 1.5 | Maleic | 1.5 | 4.20 | 3.26 | 5.39 | 1.34 | 0.86 |
| B75 | Prochlorperazine | 1.5 | Phosphoric | 1.5 | 4.20 | 4.00 | 8.11 | 1.88 | 0.75 |

**Fig. 5**

**Fig. 6**

Fig. 7

**Fig. 8**

**Fig. 8a**　　　　　　**Fig. 8b**　　　　　　**Fig. 8c**

**Fig. 9**

Fig. 10

Fig. 11

Fig. 12

Phase Stability Pre -Autoclave

**Fig. 13**

Phase Stability Post-Autoclave

Fig. 14

**Phase Stability Pre-Autoclave**

Fig. 15

Phase Stability Post-Autoclave

Fig. 16

Fig. 17

## Fig. 18

Pre-Autoclave Phase Stability

## Fig. 19

Post-Autoclave Phase Stability

**European Patent Office**

## EUROPEAN SEARCH REPORT

**Application Number**

EP 06 02 2165

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 97/34582 A (UNIV BRUXELLES [BE]; CAMU FREDERIC [BE]; ALAFANDY MOKARRAM [BE]; BRASS) 25 September 1997 (1997-09-25)<br>* the whole document *<br>* claims 1-30 * | 1-8 | INV.<br>A61K9/127 |
| X | WO 90/03808 A (LIPOSOME CO INC [US])<br>19 April 1990 (1990-04-19)<br>* the whole document *<br>* claims 1-25 * | 1-8 | |
| X | US 5 451 408 A (MEZEI MICHAEL [CA] ET AL)<br>19 September 1995 (1995-09-19)<br>* the whole document *<br>* claims 1-11 * | 1-8 | |
| X | HIROSHI KIKUCHI ET AL: "POSSIBILITY OF HEAT STERILIZATION OF LIPOSOMES"<br>CHEMICAL AND PHARMACEUTICAL BULLETIN, PHARMACEUTICAL SOCIETY OF JAPAN, TOKYO, JP,<br>vol. 39, no. 4, 1 April 1991 (1991-04-01), pages 1018-1022, XP000327266<br>ISSN: 0009-2363<br>* the whole document * | 1-8 | |
| X | ZUIDAM N J ET AL: "Sterilization of liposomes by heat treatment"<br>PHARMACEUTICAL RESEARCH, NEW YORK, NY, US, vol. 10, no. 11, 1993, pages 1591-1596, XP002969829<br>ISSN: 0724-8741<br>* the whole document * | 1-8 | TECHNICAL FIELDS SEARCHED (IPC)<br><br>A61K |

The present search report has been drawn up for all claims

| Place of search | Date of completion of the search | Examiner |
|---|---|---|
| Munich | 24 January 2007 | Felder, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 06 02 2165

This annex lists the patent family members relating to the patent documents cited in the above-mentioned European search report.
The members are as contained in the European Patent Office EDP file on
The European Patent Office is in no way liable for these particulars which are merely given for the purpose of information.

24-01-2007

| Patent document cited in search report | | Publication date | Patent family member(s) | | Publication date |
|---|---|---|---|---|---|
| WO 9734582 | A | 25-09-1997 | AT | 202278 T | 15-07-2001 |
| | | | AU | 2158097 A | 10-10-1997 |
| | | | CA | 2250073 A1 | 25-09-1997 |
| | | | DE | 69705310 D1 | 26-07-2001 |
| | | | DE | 69705310 T2 | 11-10-2001 |
| | | | EP | 0904057 A1 | 31-03-1999 |
| | | | JP | 2001500105 T | 09-01-2001 |
| | | | US | 6149937 A | 21-11-2000 |
| WO 9003808 | A | 19-04-1990 | CA | 2000176 A1 | 07-04-1990 |
| US 5451408 | A | 19-09-1995 | CA | 2119976 A1 | 24-09-1995 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4818537 A
- US 5100662 A
- US 5204112 A
- US 4804539 A
- US 5962015 A
- WO 200400246 A
- US 5554382 A
- US 5776486 A
- US 5542935 A

- US 5770222 A
- US 6071495 A
- US 6479034 B
- US 5834025 A
- US 5230899 A
- US 6424857 B
- US 5676928 A
- US RE38407 E
- WO 2004002468 A

**Non-patent literature cited in the description**

- Liposomes as Pharmaceutical Dosage Forms. **DAAN CROMMELIN.** Encyclopedia of Pharmaceutical Technology. vol. 9, 13

- Stedman's Medical Dictionary. Williams & Wilkins, 1990